# EUROPEAN PATENT APPLICATION

(11) **EP 4 210 066 A1**
(43) Date of publication of application: **12.07.2023**
(21) Application number: 21864391.4
(22) Date of filing: 02.09.2021
(51) Int. Cl.: G16H 10/60, A61B 5/02, A61B 5/022, A61B 5/1455

(54) **BIOLOGICAL INFORMATION COMPUTATION SYSTEM**

(30) Priority: 03.09.2020 JP 2020148047; 03.09.2020 JP 2020148048; 02.03.2021 JP 2021032878; 02.03.2021 JP 2021032879; 02.03.2021 JP 2021032881
(71) Applicant: SSST Co., LTD., Ueda-shi, Nagano 386-0017 (JP)
(72) Inventor: KURASAWA Shintaro, Ueda-shi, Nagano 386-0017 (JP); CHINO Shun, Ueda-shi, Nagano 386-0017 (JP)
(74) Representative: Plasseraud IP
(86) International application number: PCT/JP2021/032234
(87) International publication number: WO 2022/050336

(57) **Abstract**

[Problem]

Provided is a biometric information computing system that allows achieving a quantitative evaluation on a status of a user.

[Solution]

A biometric information computing system that calculates blood information regarding blood of a user includes obtaining means, a database, calculating means, and evaluation means. The obtaining means obtains evaluation data based on a pulse wave of the user. The database stores classification information generated using a plurality of training data. The training data is a pair of input data based on a preliminarily obtained training pulse wave and reference data including the blood information associated with the input data. The calculating means calculates the blood information for the evaluation data by referring to the database. The evaluation means generates an evaluation result regarding a status of the user based on the blood information.

## Description

### TECHNICAL FIELD

The present invention relates to a biometric information computing system.

### BACKGROUND ART

Conventionally, as a method for calculating blood information such as a blood glucose level of a user, for example, a method as disclosed in Patent Document 1 has been proposed.

A non-invasive blood glucose level measurement device disclosed in Patent Document 1 includes a pulse waveform measurement unit provided with an FBG sensor for measuring an acceleration pulse wave of a test subject, and a data-processing unit that calculates, from waveform information regarding the measured acceleration pulse wave, a blood glucose level when measurements were made of the acceleration pulse wave of the test subject, based on a predetermined correlation. The correlation is a calibration curve, which is constructed by performing a PLS regression analysis, using the blood glucose level measured by an invasive measurement method as an objective variable, and a simultaneously-measured acceleration pulse wave as an explanatory variable.

Patent Document 1: Japanese Patent No. 6544751

### DISCLOSURE OF THE INVENTION

### PROBLEMS TO BE SOLVED BY THE INVENTION

Here, with the calculation result of the blood information as disclosed in Patent Document 1, it is difficult to obtain the status of the user.

Therefore, the present invention is devised in consideration of the above-described problem, and has an object to provide a biometric information computing system capable of achieving a quantitative evaluation regarding a status of a user.

### SOLUTIONS TO THE PROBLEMS

A biometric information computing system according to the first invention is a biometric information computing system that calculates blood information regarding blood of a user, and includes: obtaining means that obtains evaluation data based on a pulse wave of the user; a database that stores classification information generated using a plurality of training data, the training data being a pair of input data based on a preliminarily obtained training pulse wave and reference data including the blood information associated with the input data; calculating means that calculates the blood information for the evaluation data by referring to the database; and evaluation means that generates an evaluation result regarding a status of the user based on the blood information.

In the biometric information computing system according to the second invention, in the first invention, the blood information includes a blood glucose level, and the evaluation means generates the evaluation result including blood glucose spike information indicating a change of the blood glucose level of the user based on a plurality of the blood glucose levels of the user calculated from the mutually different evaluation data.

In the biometric information computing system according to the third invention, in the first invention, the blood information includes a blood glucose level, and the evaluation means generates the evaluation result based on the evaluation data and the blood glucose level calculated from the evaluation data.

In the biometric information computing system according to the fourth invention, in the first invention, the blood information includes a feature regarding a composition of the blood, and the evaluation means generates the evaluation result indicating a trend of an athletic ability of the user based on a plurality of the features regarding the blood composition calculated from the mutually different evaluation data.

The biometric information computing system according to the fifth invention, in the first invention, includes aggregation means that obtains additional information indicating a feature regarding biometric information of the user and generates a comprehensive evaluation result evaluating a health condition of the user based on the evaluation result and the additional information.

In the biometric information computing system according to the sixth invention, in the first invention, the obtaining means includes obtaining additional data indicating a feature different from the evaluation data based on the pulse wave, and the calculating means includes referring to the database and generating additional information indicating a feature regarding biometric information of the user for the additional data.

In the biometric information computing system according to the seventh invention, in the first invention, the evaluation means includes: obtaining additional information indicating a feature regarding biometric information of the user; and generating the evaluation result based on a plurality of the feature data and the additional information.

In the biometric information computing system according to the eighth invention, in the first invention, the obtaining means includes obtaining preliminary evaluation data different from the evaluation data based on the pulse wave, the classification information includes a plurality of pieces of attribute-based classification information calculated using the mutually different training data, and the calculating means includes: selection means that refers to the preliminary evaluation data and selects first classification information among the plurality of pieces of attribute-based classification information; and attribute-based calculating means that refers to the first classification information and calculates the blood information for the evaluation data.

### EFFECTS OF THE INVENTION

According to the first invention to the eighth invention, the calculating means calculates the blood information of the user for the evaluation data. The evaluation means generates the evaluation result on the status of the user based on the blood information. Accordingly, the quantitative evaluation regarding the status of the user can be achieved.

### BRIEF DESCRIPTION OF THE DRAWINGS

Fig. 1 is a schematic diagram illustrating an example of a biometric information computing system in a first embodiment.
Fig. 2A and Fig. 2B are schematic diagrams illustrating examples of an operation of the biometric information computing system in the first embodiment.
Fig. 3A is a schematic diagram illustrating an example of classification information, and Fig. 3B and Fig. 3C are schematic diagrams illustrating examples of processing on sensor data.
Fig. 4A is a schematic diagram illustrating an example of a configuration of a biometric information computing device, and Fig. 4B is a schematic diagram illustrating an example of functions of the biometric information computing device.
Fig. 5A and Fig. 5B are schematic diagrams illustrating an example of a sensor.
Fig. 6 is a flowchart illustrating an example of the operation of the biometric information computing system in the first embodiment.
Fig. 7 is a schematic diagram illustrating an example of an operation of a biometric information computing system in a second embodiment.
Fig. 8 is a schematic diagram illustrating a modification of the operation of the biometric information computing system in the second embodiment.
Fig. 9 is a schematic diagram illustrating an example of an operation of a biometric information computing system in a third embodiment.
Fig. 10A and Fig. 10B are schematic diagrams illustrating examples of processing for calculating additional information for sensor data.
Fig. 11 is a schematic diagram illustrating an example of an operation of a biometric information computing system in the third embodiment.
Fig. 12 is a schematic diagram illustrating an example of an operation of a biometric information computing system in a fourth embodiment.
Fig. 13 is a schematic diagram illustrating an example of an operation of a biometric information computing system in a fifth embodiment.
Fig. 14 is a schematic diagram illustrating a classification example of data corresponding to an acceleration pulse wave.
Fig. 15 is a schematic diagram illustrating a classification example of data corresponding to a velocity pulse wave.
Fig. 16 is a schematic diagram illustrating a modification of the operation of the biometric information computing system in the fifth embodiment.
Fig. 17A and Fig. 17B are schematic diagrams illustrating examples of an operation of a biometric information computing system in a sixth embodiment.
Fig. 18 is a schematic diagram illustrating an example of an operation of a biometric information computing system in a seventh embodiment.
Fig. 19 is a schematic diagram illustrating a blood glucose trend.
Fig. 20 is a schematic diagram illustrating an example of an operation of a biometric information computing system in an eighth embodiment.
Fig. 21 is a schematic diagram illustrating another example of the operation of the biometric information computing system in the eighth embodiment.
Fig. 22 is a schematic diagram illustrating an example of an operation of a biometric information computing system in a ninth embodiment.
Fig. 23 is a schematic diagram illustrating an example of an operation of a biometric information computing system in a tenth embodiment.
Fig. 24A and Fig. 24B are schematic diagrams illustrating examples of an operation of a biometric information computing system in an eleventh embodiment.
Fig. 25A and Fig. 25B are schematic diagrams illustrating an example of a process of generating an evaluation result.

### DESCRIPTION OF PREFERRED EMBODIMENTS

The following describes examples of a biometric information computing system according to embodiments of the present invention by referring to the drawings.

The biometric information computing system 100 is used for calculating blood information regarding blood of a user, and generates an evaluation result on a status of the user based on the calculated blood information. As the blood information, for example, information that can be calculated based on a pulse wave is usable, and for example, a blood glucose level is included, additionally, a feature regarding blood composition is included. As the blood information, the status of the user indicates, for example, a change of the blood glucose level of the user, and additionally, indicates an athletic ability of the user and the health condition of the user.

In the following description, in the first embodiment to the fifth embodiment, a biometric information computing system 100 when the blood information includes the blood glucose level and the status of the user indicates the change of the blood glucose level of the user will be described. In the sixth embodiment to the tenth embodiment, a biometric information computing system 100 when the blood information includes the blood glucose level and the status of the user indicates the health condition of the user will be described. In the eleventh embodiment to the twelfth embodiment, a biometric information computing system 100 when the blood information includes the feature regarding the blood composition and the status of the user indicates the athletic ability of the user will be described.

### (First Embodiment: Biometric Information Computing System 100)

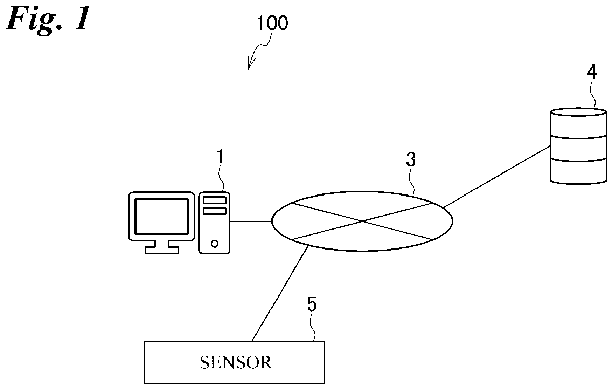

Fig. 1 is a schematic diagram illustrating an example of a biometric information computing system 100 in the first embodiment.

The biometric information computing system 100 according to this embodiment is configured to calculate the blood glucose level of the user, and evaluate the change of the user's blood glucose level based on a plurality of the calculated blood glucose levels.

The change of user's blood glucose level can be indicated by, for example, blood glucose spike information. The blood glucose spike information indicates, for example, an average value, a width of value (for example, intensity of blood glucose spike indicated by a difference between the maximum value and the minimum value), a median, or the like relative to the plurality of blood glucose levels.

Additionally, the blood glucose spike information indicates, for example, a value obtained from a change in time series in a plurality of blood glucose levels, and indicates, for example, a feature of temporal change derived using differentiation or a feature of a peak shape derived using integration. The "blood glucose spike" includes a publicly known definition indicating the change of blood glucose level.

In the biometric information computing system 100, an evaluation result including the above-described blood glucose spike information can be generated. The evaluation result may include not only the value and the feature indicated by the blood glucose spike information but also, for example, a health condition or the like evaluated based on the blood glucose spike information.

For example, as illustrated in Fig. 1, the biometric information computing system 100 includes a biometric information computing device 1, and for example, may include at least any of a sensor 5 and a server 4. The biometric information computing device 1 is connected to the sensor 5 and the server 4 via, for example, a communications network 3.

The biometric information computing system 100 calculates the blood glucose level from evaluation data based on a pulse wave of a user. The biometric information computing system 100 generates an evaluation result including the blood glucose spike information based on, for example, a plurality of blood glucose levels generated in time series or at any timings.

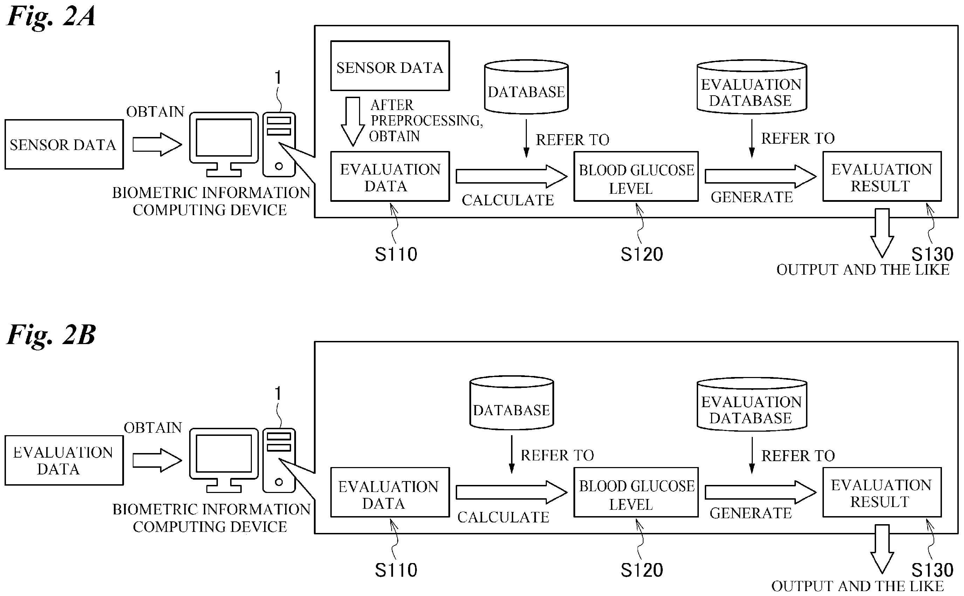

In the biometric information computing system 100, for example, as illustrated in Fig. 2A, the biometric information computing device 1 obtains sensor data generated by the sensor 5 or the like. Then, the biometric information computing device 1 performs preprocessing, such as a filter process, on the obtained sensor data to obtain the evaluation data.

The biometric information computing device 1 calculates the blood glucose level for the evaluation data. Then, the biometric information computing device 1 generates the evaluation result based on the plurality of blood glucose levels generated from the mutually different evaluation data. Therefore, regardless of individual differences of users, the evaluation result to the change of the blood glucose level can be obtained. Accordingly, the quantitative evaluation regarding the change of the blood glucose level can be achieved.

Here, the biometric information computing device 1 refers to the database in the calculation of the blood glucose level for the evaluation data. The database stores classification information generated using a plurality of training data.

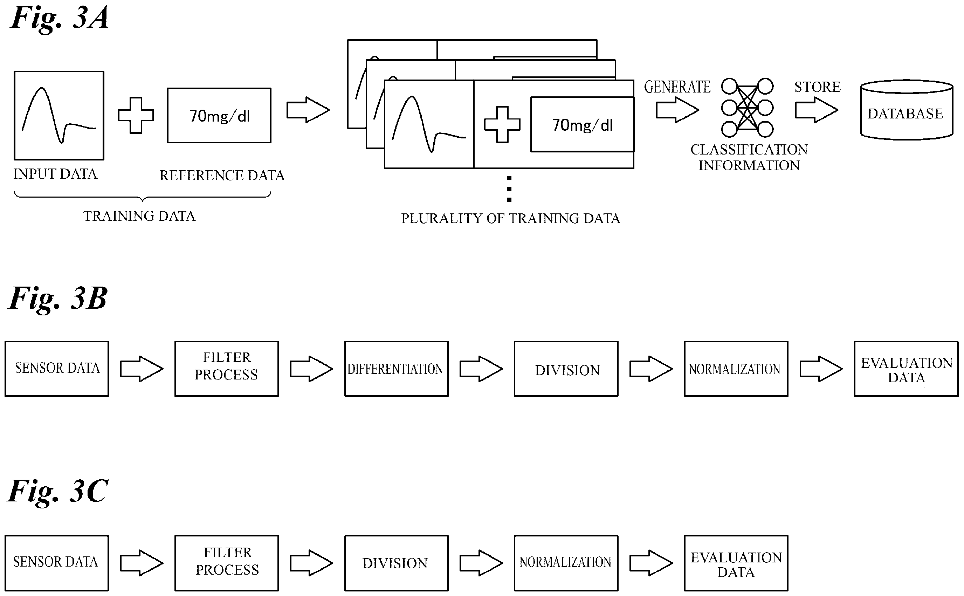

For example, as illustrated in Fig. 3A, the classification information is generated using a plurality of training data each of which is a pair of input data based on a training pulse wave obtained in the past and reference data including the past blood glucose level associated with the input data. Therefore, in the calculation of the blood glucose level, the blood glucose level can be quantitatively calculated based on the input data proven in the past and the output data. This allows attempting the improvement of the accuracy in the calculation of the blood glucose level.

The biometric information computing device 1 outputs the generated evaluation results to, for example, a display or the like. The evaluation result includes the blood glucose spike information indicating the change of the user's blood glucose level, and includes, for example, the blood glucose spike information indicated by a numerical value. The evaluation result may include, for example, an index indicating a possibility of diabetes, information on recommended meals, or information on exercise.

In the biometric information computing system 100, for example, as illustrated in Fig. 2B, the evaluation data may be obtained from the sensor 5 or the like. In this case, the preprocessing of obtaining the evaluation data from the sensor data is performed by the sensor 5 or the like.

### <Sensor Data>

The sensor data includes data indicating the feature of the user's pulse wave, and may include, for example, data (noise) indicating a feature other than the pulse wave. The sensor data is data indicating an amplitude to a measurement time, and by performing a filter process depending on the usage or the generation condition of the sensor data, data corresponding to an acceleration pulse wave, a velocity pulse wave, or the like can be obtained from the sensor data.

The sensor data can be generated with a publicly known sensor, such as a strain sensor, a gyro sensor, a photoplethysmogram (PPG) sensor, and a pressure sensor. The sensor data may be a digital signal, and additionally, for example, an analog signal. The measurement time in generating the sensor data is a measurement time by, for example, 1 to 20 cycles of the pulse wave, and can be appropriately set depending on the condition such as a processing method of the sensor data and a data communication method.

### <Evaluation Data>

The evaluation data indicates data for calculating the blood glucose level. The evaluation data indicates, for example, data corresponding to the acceleration pulse wave based on the user's pulse wave, and indicates an amplitude to a specific cycle (for example, one cycle).

The evaluation data is obtained through performing a process (preprocessing) to the sensor data by the biometric information computing device 1 or the like. For example, as illustrated in Fig. 3B and Fig. 3C, by performing a plurality of processes on the sensor data, the evaluation data can be obtained. Details of the respective processes will be described later.

### <Database>

The database is mainly used for calculating the user's blood glucose level for the evaluation data. The database stores one or more pieces of the classification information, and additionally, may store, for example, the plurality of training data used for generating the classification information.

The classification information is a function indicating, for example, a correlation between preliminarily obtained past evaluation data (input data) and reference data including the blood glucose level. The classification information indicates a calibration model generated based on a result of an analysis performed by, for example, a regression analysis having the input data as an explanatory variable and the reference data as an objective variable. For the classification information, for example, the calibration model can be regularly updated, and additionally, a plurality of calibration models generated for respective pieces of attribute information such as a gender, an age, and an exercise content of the user may be included.

As a method of the regression analysis used in the generation of the classification information, for example, PLS (Partial Least Squares) regression analysis, a regression analysis using SIMCA (Soft Independent Modeling of Class Analogy) method in which a principal component analysis is performed for each class to obtain a principal component model, or the like can be used.

The classification information may include, for example, a trained model generated through machine learning using a plurality of training data. The trained model indicates, for example, a neural network model such as CNN (Convolutional Neural Network), and additionally, indicates SVM (Support vector machine) or the like. As the machine learning, for example, deep learning can be used.

As the input data, data of the same kind as the evaluation data is used, and for example, past evaluation data in which the corresponding blood glucose level is clear is indicated. For example, the sensor 5 or the like is worn by an examinee, and sensor data (training sensor data) indicating the feature of the training pulse wave is generated. Then, by performing a process on the training sensor data, the input data can be obtained. The input data is obtained from the user of the biometric information computing system 100, and additionally, for example, may be obtained from another user. That is, the above-described examinee is a user of the biometric information computing system 100, and additionally, may be targeted to those other than the user, and may be a large number of specified or unspecified people.

The input data is preferably obtained, for example, with the contents similar to the type of the sensor 5 or the like used in obtaining the evaluation data, the generation condition of the sensor data, and the process condition for the sensor data. For example, by making the above-described three contents uniform, the accuracy in calculating the blood glucose level can be tremendously improved.

The reference data includes the blood glucose level of the examinee measured using a measuring device or the like. For example, when the sensor 5 or the like is worn by the examinee to generate the training sensor data, by measuring the blood glucose level of the examinee, the reference data associated with the input data can be obtained. In this case, while a timing of measuring the blood glucose level is preferably simultaneous with a timing of generating the training sensor data, the timing of measuring the blood glucose level may be a timing, for example, before or after about one to 10 minutes. As the measuring device for measuring the blood glucose level, for example, a publicly known blood glucose meter such as FreeStyle Precision Pro (manufactured by Abbott Japan Co., Ltd.) is used.

### <Evaluation Result>

The evaluation result includes the blood glucose spike information indicating the change of the user's blood glucose level. The evaluation result includes, for example, a plurality of blood glucose levels, and additionally, may include difference values between the plurality of blood glucose levels or a degree of difference between the plurality of blood glucose levels and a predetermined threshold. By outputting the evaluation result, the change of the user's blood glucose level can be obtained.

### <Biometric Information Computing Device 1>

The biometric information computing device 1 indicates, for example, an electronic device such as a personal computer (PC), a mobile phone, a smartphone, a tablet terminal, and a wearable device, and indicates, for example, an electronic device communicatable via the communications network 3 based on the operation of the user. The biometric information computing device 1 may include the sensor 5. The following describes an example of a case where a PC is used as the biometric information computing device 1.

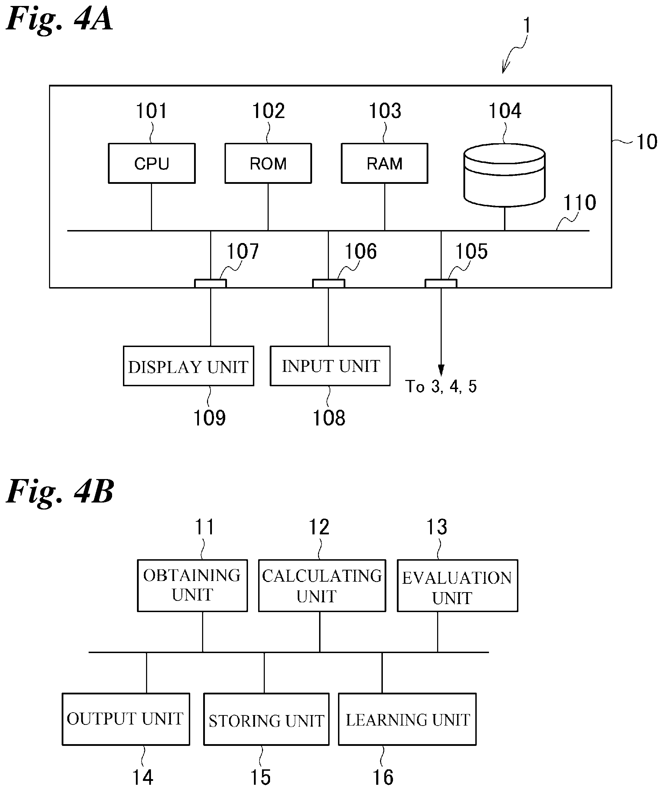

Fig. 4A is a schematic diagram illustrating an example of a configuration of the biometric information computing device 1, and Fig. 4B is a schematic diagram illustrating an example of functions of the biometric information computing device 1.

For example, as illustrated in Fig. 4A, the biometric information computing device 1 includes a housing 10, a CPU (Central Processing Unit) 101, a ROM (Read Only Memory) 102, a RAM (Random Access Memory) 103, a storage unit 104, and I/Fs 105 to 107. The configurations 101 to 107 are mutually connected via an internal bus 110.

The CPU 101 controls the entire biometric information computing device 1. The ROM 102 stores operation codes of the CPU 101. The RAM 103 is a work area used in the operation of the CPU 101. The storage unit 104 stores various kinds of information such as the database and the evaluation data. As the storage unit 104, for example, a data storage device such as an SSD (Solid State Drive) or the like is used in addition to an HDD (Hard Disk Drive). For example, the biometric information computing device 1 may include a not illustrated GPU (Graphics Processing Unit).

The I/F 105 is an interface for transmitting and receiving various kinds of information with the server 4, the sensor 5, and the like via the communications network 3 as necessary. The I/F 106 is an interface for transmitting and receiving the information with an input unit 108. As the input unit 108, for example, a keyboard is used, and the user or the like of the biometric information computing device 1 inputs various kinds of information, control commands of the biometric information computing device 1, or the like via the input unit 108. The I/F 107 is an interface for transmitting and receiving various kinds of information with a display unit 109. The display unit 109 displays various kinds of information, the evaluation result, or the like stored in the storage unit 104. As the display unit 109, a display is used, and for example, in a case of a touch panel type, the display unit 109 is provided integrally with the input unit 108.

Fig. 4B is a schematic diagram illustrating an example of functions of the biometric information computing device 1. The biometric information computing device 1 includes an obtaining unit 11, a calculating unit 12, an evaluation unit 13, an output unit 14, and a storing unit 15, and may include, for example, a learning unit 16. The functions illustrated in Fig. 4B are each achieved by executing programs stored in the storage unit 104 or the like by the CPU 101 with the RAM 103 as the work area.

### <Obtaining Unit 11>

The obtaining unit 11 obtains evaluation data based on the user's pulse wave. For example, after obtaining the sensor data from the sensor 5 or the like, the obtaining unit 11 performs a process on the sensor data to obtain the evaluation data.

For example, as illustrated in Fig. 3B, the obtaining unit 11 performs a filtering process (filter process) on the obtained sensor data. In the filter process, for example, a bandpass filter of 0.5 to 5.0 Hz is used. Accordingly, the obtaining unit 11 extracts data (pulse wave data) corresponding to the user's pulse wave. The pulse wave data indicates, for example, data corresponding to the velocity pulse wave. The pulse wave data may indicate data corresponding to, for example, the acceleration pulse wave or the plethysmogram, and can be appropriately set depending on the type or the usage of the sensor. The filter range of bandpass filter can be appropriately set depending on the usage.

The obtaining unit 11 performs, for example, a differential process on the pulse wave data. For example, when the differential process is performed to the pulse wave data corresponding to the velocity pulse wave, the obtaining unit 11 obtains data (differential data) corresponding to the acceleration pulse wave. In the differential process, a two time differentiation may be performed in addition to a one time differentiation.

The obtaining unit 11 performs, for example, a dividing process on the differential data. In the dividing process, for example, differential data corresponding to the acceleration pulse wave of a plurality of cycles is divided into data (divided data) corresponding to the acceleration pulse waves of respective cycles. Therefore, for example, by performing the differential process on one differential data, the obtaining unit 11 can obtain a plurality of divided data. In the dividing process, the differential data can be divided for each of any cycles (for example, positive number multiple of cycle) depending on the usage.

For example, in the dividing process, the data amounts of the divided respective divided data are mutually different in some cases. In this case, the obtaining unit 11 may identify the divided data with the least data amount, and may perform reduction of the data amount (trimming) to the other divided data. This allows making the data amounts of the respective divided data uniform, thus facilitating data comparison between the respective divided data.

Additionally, for example, a normalization process may be performed to a value corresponding to a time axis of the divided data. In the normalization process, for example, the normalization in which the minimum value of the value corresponding to the time axis is set to 0 and the maximum value is set to 1 is performed. Accordingly, the data comparison between the respective divided data is facilitated.

The obtaining unit 11 may provide the divided data by, for example, calculating a mean of a plurality of divided data to which the reduction of the data amount or the normalization has been performed.

The obtaining unit 11 performs the normalization process on the divided data. In the normalization process, data of being normalized (normalized data) is generated to a value corresponding to the amplitude. In the normalization process, for example, the normalization in which the lowest value of the amplitude is set to 0 and the highest value of the amplitude is set to 1 is performed. The obtaining unit 11 obtains, for example, the normalized data as evaluation data. In this case, as the evaluation data, data corresponding to the acceleration pulse wave of the user is obtained.

The obtaining unit 11 sequentially performs the above-described respective processes, and additionally, for example, as illustrated in Fig. 3C, the obtaining unit 11 does not need to perform the differential process. In this case, as the evaluation data, data corresponding to the velocity pulse wave of the user is obtained.

The obtaining unit 11 may perform, for example, only a part of the above-described processes. In this case, the obtaining unit 11 may obtain any of the pulse wave data, the differential data, the divided data, the trimmed divided data, and the divided data in which the value corresponding to the time axis has been normalized as the evaluation data, and the setting can be appropriately made depending on the usage.

### <Calculating Unit 12>

The calculating unit 12 calculates the user's blood glucose level for the evaluation data with reference to the database. The calculating unit 12 refers to, for example, the classification information stored in the database, and calculates the blood glucose level for the evaluation data. The calculating unit 12 generates a plurality of the blood glucose levels for the mutually different evaluation data.

The blood glucose level calculated by the calculating unit 12 is calculated as data of the same kind as the reference data. The blood glucose level is calculated as data the same as or similar to the reference data with reference to the classification information. In the biometric information computing system 100, for example, a plurality of evaluation data are obtained along any time series, and a plurality of blood glucose levels for the respective evaluation data are calculated. In the biometric information computing system 100, for example, a plurality of evaluation data may be obtained for each of any timings, such as before and after a meal or when the quantity of exercise has changed, and a plurality of blood glucose levels for the respective evaluation data may be calculated.

### <Evaluation Unit 13>

The evaluation unit 13 generates the evaluation result including the blood glucose spike information indicating the change of the user's blood glucose level based on the plurality of blood glucose levels calculated from the mutually different evaluation data. The evaluation unit 13 generates the evaluation result transformed into a format in which the user can understand the blood glucose spike information, for example, using a display format preliminarily stored in the storage unit 104 or the like.

The evaluation unit 13 may calculate, for example, a difference between the minimum value and the maximum value of the plurality of blood glucose levels as the blood glucose spike information. In this case, the change amount of the blood glucose level can be used as an evaluation target. Accordingly, for example, the feature of potential diabetics can be easily separated from the feature of diabetics.

The evaluation unit 13 calculates, for example, a value obtained by dividing a difference between two blood glucose levels by a difference of measurement time as the blood glucose spike information, thus generating the evaluation result including the blood glucose spike information. In this case, the change of the blood glucose level that differs for each user can be easily obtained. For example, the evaluation unit 13 may refer to a reference value preliminarily stored in the storage unit 104 or the like, generate the evaluation result indicating the fact of being in a healthy condition when the blood glucose spike information is smaller than the reference value, and generate the evaluation result indicating the fact of being in a possibly diabetic condition when the blood glucose spike information is equal to or more than the reference value.

The evaluation unit 13 may generate the evaluation result from a plurality of blood glucose levels, for example, by referring to an evaluation database. The evaluation database is stored in, for example, the storage unit 104.

In the evaluation database, for example, similarly to the above-described database, evaluation classification information for generating the evaluation result for the plurality of blood glucose levels may be stored. In the evaluation database, one or more pieces of the evaluation classification information are stored, and additionally, for example, a plurality of evaluation training data used for generating the evaluation classification information may be stored.

The evaluation classification information is, for example, a function indicating a correlation between a preliminarily obtained plurality of past blood glucose levels (evaluation input data) and evaluation reference data associated with the evaluation input data. The evaluation reference data indicates the trend of the change of the user's blood glucose level, and indicates, for example, the blood glucose spike information generated in the past. The evaluation classification information is generated using a plurality of evaluation training data each of which is a pair of the evaluation input data and the evaluation reference data.

The evaluation classification information indicates a calibration model generated based on a result of an analysis performed by, for example, the above-described regression analysis having the evaluation input data as an explanatory variable and the evaluation reference data as an objective variable. For the evaluation classification information, for example, the calibration model (evaluation calibration model) can be regularly updated, and additionally, a plurality of evaluation calibration models generated for respective pieces of attribute information such as a gender, an age, an exercise content, and the like of the user may be included. The evaluation classification information may include, similarly to the above-described classification information, for example, a trained model (evaluation trained model) generated by machine learning using a plurality of evaluation training data.

The evaluation unit 13 calculates the blood glucose spike information from, for example, a time series change of a plurality of blood glucose levels, thus generating the evaluation result. In this respect, a section of the time series or the number of blood glucose levels can be appropriately set. Therefore, the evaluation result considering the feature of the shape in addition to the intensity of the blood glucose spike can be generated.

The evaluation unit 13 may calculate, for example, data in which a plurality of blood glucose levels are associated with obtaining times of the evaluation data as the blood glucose spike information. In this case, as the evaluation result, information indicative of the trend of the blood glucose level can be included.

### <Output Unit 14>

The output unit 14 outputs an evaluation result. The output unit 14 outputs the evaluation result to the display unit 109, and additionally, may output the evaluation result to, for example, the sensor 5 or the like.

### <Storing Unit 15>

The storing unit 15 retrieves various kinds of data such as a database stored in the storage unit 104 as necessary. The storing unit 15 stores the various kinds of data obtained or generated by the configurations 11 to 14, and 16 in the storage unit 104 as necessary.

### <Learning Unit 16>

The learning unit 16 generates the classification information, for example, using a plurality of training data. The learning unit 16 may, for example, obtain new training data and update the existing classification information.

The learning unit 16 generates the evaluation classification information, for example, using a plurality of evaluation training data. The learning unit 16 may, for example, obtain new evaluation training data and update the existing evaluation classification information.

In the biometric information computing system 100, when the classification information and the evaluation classification information are used, for example, the classification information may be updated without updating the evaluation classification information depending on the kind of the evaluation data. In this case, since it is not necessary to prepare additional evaluation training data, the cost reduction and the significant reduction of update time can be achieved.

### <Communications Network 3>

The communications network 3 is a publicly known Internet network or the like in which the biometric information computing device 1, the server 4, and the sensor 5 are connected via a communication line. The communications network 3 may be established by a LAN (Local Area Network) or the like when the biometric information computing system 100 is operated in a certain narrow area. The communications network 3 may be established by what is called an optical fiber communications network. The communications network 3 is not limited to a wired communication network, may be achieved by a wireless communications network, and can be appropriately set depending on the usage.

### <Server 4>

The server 4 accumulates information transmitted via the communications network 3. The server 4 transmits the accumulated information to the biometric information computing device 1 via the communications network 3 based on the request from the biometric information computing device 1.

The server 4 may be connected to, for example, a plurality of biometric information computing devices 1, obtain the various kinds of information such as the evaluation result from each of the biometric information computing devices 1, and collectively store the various kinds of information. The server 4 may include at least a part of the functions among the above-described functions included in the biometric information computing device 1. The server 4 may store the above-described database or the like stored in the biometric information computing device 1.

### <Sensor 5>

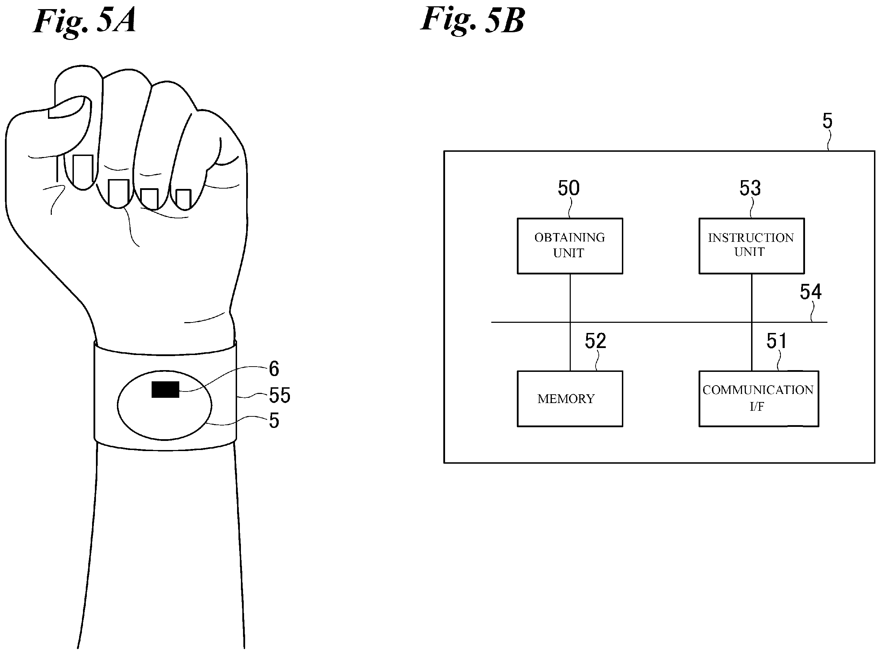

The sensor 5 generates the sensor data. For example, as illustrated in Fig. 5A, the sensor 5 includes a detecting unit 6. The sensor 5 is worn at a position at which the user's pulse wave can be detected via the detecting unit 6, and for example, fixed to a wristband 55.

As the detecting unit 6, a publicly known detecting device that can detect the user's pulse wave is used. As the detecting unit 6, for example, at least any of a strain sensor such as a fiber bragg grating (FBG) sensor, a gyro sensor, one or more electrodes for measuring a pulse wave signal, a photoplethysmogram (PPG) sensor, a pressure sensor, and a photo-detection module is used. For example, a plurality of the detecting units 6 may be provided.

The sensor 5 may be embedded in clothing. The user wearing the sensor 5 may be not only a human but also a pet such as a dog and a cat, and for example, may be a domestic animal such as a cow and a pig, a farmed fish, or the like.

For example, as illustrated in Fig. 5B, the sensor 5 includes an obtaining unit 50, a communication I/F 51, a memory 52, and an instruction unit 53, and the configurations are mutually connected by an internal bus 54.

The obtaining unit 50 measures the user's pulse wave via the detecting unit 6, and generates the sensor data. The obtaining unit 50 transmits, for example, the generated sensor data to the communication I/F 51 or the memory 52.

The communication I/F 51 transmits the various kinds of data such as sensor data to the biometric information computing device 1 and the server 4 via the communications network 3. The communication I/F 51 includes a line control circuit for connecting to the communications network 3, a signal conversion circuit for performing a data communication with the biometric information computing device 1 and the server 4, and the like. The communication I/F 51 performs conversion processing to various kinds of instructions from the internal bus 54 and delivers these to the communications network 3 side, and when data from the communications network 3 is received, the communication I/F 51 performs predetermined conversion processing to the data, and transmits it to the internal bus 54.

The memory 52 stores various kinds of data such as sensor data transmitted from the obtaining unit 50. For example, the memory 52 receives an instruction from another terminal device connected via the communications network 3, thereby transmitting the stored various kinds of data such as sensor data to the communication I/F 51.

The instruction unit 53 includes an operating button, a keyboard, or the like for obtaining the sensor data, and for example, includes a processor such as a CPU. When an instruction to obtain the sensor data is accepted, the instruction unit 53 notifies the obtaining unit 50 of it. The obtaining unit 50 that has received the notification obtains the sensor data. For example, as illustrated in Fig. 3B and Fig. 3C, the instruction unit 53 may perform a process for obtaining the evaluation data from the sensor data.

Here, as an example of obtaining the sensor data, a case of using an FBG sensor will be described.

The FBG sensor is one in which diffraction grating structures are formed at predetermined intervals in one optical fiber. The FBG sensor has a feature of, for example, a sensor part length of 10 mm, a wavelength resolution of ±0.1 pm, a wavelength range of 1550 ± 0.5 nm, a fiber diameter of 145 µm, and a core diameter of 10.5 µm. The measurement can be performed with the FBG sensor as the above-described detecting unit 6 in contact with the user's skin.

For example, as a light source used for the optical fiber, an ASE (Amplified Spontaneous Emission) light source with the wavelength range of 1525 to 1570 nm is used. An emitted light from the light source enters the FBG sensor via a circulator. A reflected light from the FBG sensor is guided to a Mach-Zehnder interferometer via the circulator, and an output light from the Mach-Zehnder interferometer is detected by an optical detector. The Mach-Zehnder interferometer is one for creating an interference light by splitting an optical path into two optical paths with an optical path difference by a beam splitter and superimposing the two optical paths into one again by the beam splitter. To provide the optical path difference, for example, the length of one optical fiber may be lengthened. Since interference fringes are produced in a coherent light corresponding to the optical path difference, by measuring the pattern of the interference fringe, the strain change in the FBG sensor, that is, the pulse wave can be detected. The obtaining unit 50 generates the sensor data based on the detected pulse wave. Accordingly, the sensor data is obtained.

The optical fiber sensor system that detects the waveform of the pulse wave by detecting the strain amount of the FBG sensor includes, in addition to the light source whose light is entered in the FBG sensor, an optical system such as an ASE light source with a wide bandwidth, a circulator, a Mach-Zehnder interferometer, and a beam splitter, a light receiving sensor included in the optical detector, and an analysis method for analyzing a wavelength shift amount. The optical fiber sensor system can be used by selecting the light source and bandwidth light depending on the characteristics of the FBG sensor to be used, and for the analysis method such as a wave detection method, various kinds of methods are employable.

### (First Embodiment: Operation of Biometric Information Computing System 100)

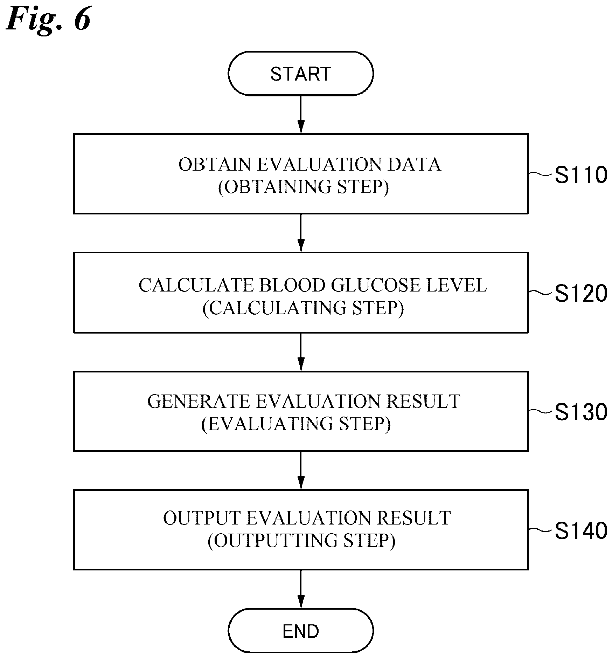

Next, an example of the operation of the biometric information computing system 100 according to the embodiment will be described. Fig. 6 is a flowchart illustrating an example of the operation of the biometric information computing system 100 in this embodiment.

The biometric information computing system 100 is executed, for example, via a biometric information computing program installed in the biometric information computing device 1. That is, the user operates the biometric information computing device 1 or the sensor 5, thereby being able to obtain an evaluation result indicating the change of the user's blood glucose level or the like from the sensor data through the biometric information computing program installed in the biometric information computing device 1.

The operation of the biometric information computing system 100 includes an obtaining step S110, a calculating step S120, and an evaluating step S130, and may include, for example, an outputting step S140.

### <Obtaining Step S110>

The obtaining step S110 obtains the evaluation data based on the user's pulse wave. For example, the obtaining unit 50 of the sensor 5 measures the user's pulse wave via the detecting unit 6 to generate the sensor data. The obtaining unit 50 transmits the sensor data to the biometric information computing device 1 via the communication I/F 51 and the communications network 3. The obtaining unit 11 of the biometric information computing device 1 receives the sensor data from the sensor 5.

The obtaining unit 11 performs, for example, the process illustrated in Fig. 3B on the sensor data, thereby obtaining the evaluation data. The obtaining unit 11 stores the obtained evaluation data in the storage unit 104, for example, via the storing unit 15. The condition such as a frequency of obtaining the sensor data from the sensor 5 by the obtaining unit 11 can be appropriately set depending on the usage. For example, the obtaining unit 11 obtains the evaluation data in a preliminarily set cycle. In this case, since the arithmetic processing in generating the evaluation result can be simplified, the improvement of processing speed can be attempted.

### <Calculating Step S120>

Next, the calculating step S120 refers to the database, and calculates the blood glucose level of the user for the evaluation data. For example, the calculating unit 12 refers to the classification information, and calculates the blood glucose level for the evaluation data. The calculating unit 12 stores the calculated blood glucose level in the storage unit 104, for example, via the storing unit 15. The blood glucose level indicates a specific value, and additionally, for example, an error range (for example, "xx ± 5 mg/dl") may be calculated.

### <Evaluating Step S130>

Next, the evaluating step S130 generates the evaluation result including the blood glucose spike information based on the plurality of blood glucose levels generated from the mutually different evaluation data. For example, the evaluation unit 13 obtains the plurality of blood glucose levels generated by the calculating unit 12. The evaluation unit 13 calculates the blood glucose spike information from the plurality of blood glucose levels using a preliminarily set function or the like, and additionally, for example, may generate the evaluation result by referring to the above-described evaluation database.

### <Outputting Step S140>

Next, for example, the outputting step S140 may output the evaluation result. For example, the output unit 14 outputs the evaluation result to the display unit 109.

Accordingly, the operation of the biometric information computing system 100 ends. The frequency and the order of executing the steps can be appropriately set depending on the usage.

Here, examples of the index for evaluating the health condition of the user include a degree of the blood glucose spike indicating the rapid change of the blood glucose level. While the blood glucose spike has been known as the feature specific to the potential diabetics, it is difficult to quantitatively evaluate the timing or the degree of the occurrence of the blood glucose spike due to the individual difference. Additionally, since the blood glucose level of the diabetic tends to be constantly high, the blood glucose level is similar to the occurrence of the blood glucose spike. Therefore, it is difficult to determine whether the blood glucose level measured by the user as necessary is to be classified into a value at the occurrence of the blood glucose spike or a value specific to the diabetic, and this possibly causes a subjective view to be included in the determination whether the user belongs to the potential diabetics or the diabetic. In view of this, it is desired to achieve the quantitative evaluation regarding the change of the blood glucose level.

In this respect, the conventional techniques remain in capable of calculating the blood glucose level by non-invasion, and have difficulty in leading to finding and solving the above-described problem on the blood glucose level change.

In contrast, according to this embodiment, the calculating unit 12 calculates the blood glucose level of the user for the evaluation data. The evaluation unit 13 generates the evaluation result including the blood glucose spike information indicating the change of the user's blood glucose level based on the plurality of blood glucose levels calculated from the mutually different evaluation data. Therefore, the evaluation result for the change of the blood glucose level can be obtained regardless of the individual difference of the user. Accordingly, the quantitative evaluation regarding the change of the blood glucose level can be achieved.

According to this embodiment, the calculating unit 12 refers to the database, and calculates the user's blood glucose level for the evaluation data. The database stores the classification information generated using a plurality of training data. Therefore, in the calculation of the blood glucose level, the quantitative blood glucose level based on the data proven in the past can be calculated. Accordingly, the accuracy improvement in the calculation of the blood glucose level can be attempted.

According to this embodiment, the evaluation unit 13 calculates the blood glucose spike information from the time series change of the plurality of blood glucose levels, thus generating the evaluation result. Therefore, the evaluation result considering the feature of the shape in addition to the intensity of the blood glucose spike can be generated. Accordingly, the accuracy in the evaluation regarding the change of the blood glucose level can be improved.

### (Second Embodiment: Biometric Information Computing System 100)

Next, an example of a biometric information computing system 100 in the second embodiment will be described. The difference between the above-described embodiment and the second embodiment is that additional information is used. For the contents similar to the above-described embodiment, the explanation will be omitted.

In the biometric information computing system 100 according to this embodiment, for example, an aggregating step S150 is performed after the above-described evaluating step S130, and an outputting step S140 is performed after the aggregating step S150.

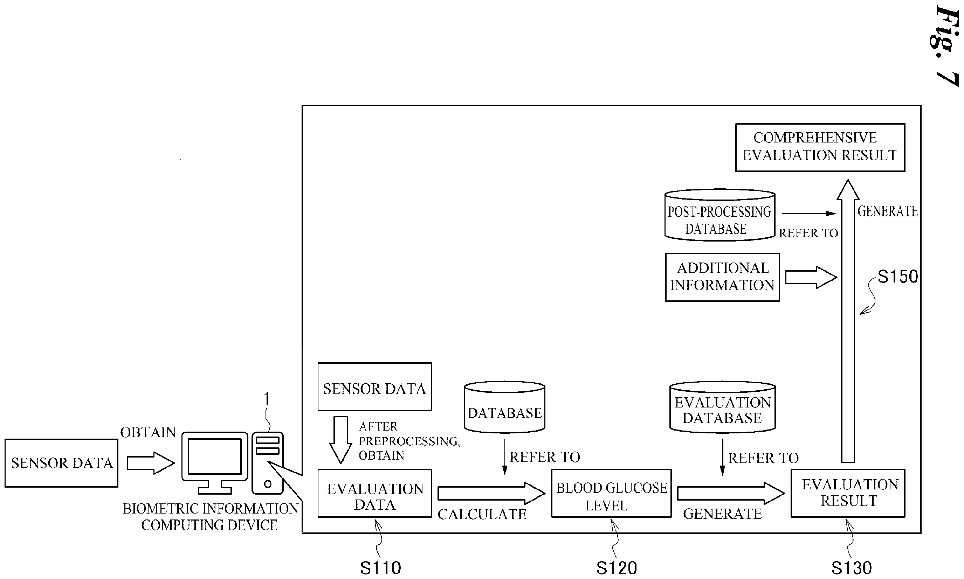

For example, as illustrated in Fig. 7, the aggregating step S150 obtains additional information, and generates comprehensive evaluation result evaluating the health condition of the user based on the evaluation result and the additional information. The aggregating step S150 can be executed by, for example, an aggregation unit included in the evaluation unit 13.

The additional information indicates the feature regarding biometric information of the user, and includes, for example, at least any of the biometric information that can be calculated based on the pulse wave, such as a pulse rate, a respiratory rate, a blood pressure, a lactate level, a feature of blood carbon dioxide, an oxygen saturation, a vascular age, a stress level, and a degree of diabetes of the user. The additional information can be used, for example, in estimating the exercise condition or the health condition of the user. The additional information includes, for example, the attribute information such as a gender and an age of the user as the feature regarding the biometric information of the user, and additionally, may include information on exercise that possibly affects the biometric information of the user, such as an exercise content and an athletic event of the user. As the biometric information, for example, information that can be calculated based on the pulse wave is usable.

The "feature of blood carbon dioxide" indicates a level of carbon dioxide contained in blood. As the feature of blood carbon dioxide, for example, besides a value of a blood carbon dioxide partial pressure (PaCO₂) being used, a dissolved concentration of blood carbon dioxide and a concentration of bicarbonate (HCO₃⁻) contained in blood may be used or a value considering pH of blood may be used depending on the situation.

The additional information is measured, for example, using a publicly known measuring device, and any data format is usable. For example, in the case of measuring the feature of the blood carbon dioxide concentration, as the measuring device, a device such as transcutaneous blood gas monitor TCM5 (manufactured by Radiometer Basel) is used. For example, when a blood lactate level is measured, as the measuring device, a publicly known device such as Lactate Pro 2 (manufactured by ARKRAY, Inc.) is used. For example, when an oxygen saturation is measured, as the measuring device, a publicly known device such as PULSOX-Neo (manufactured by KONICA MINOLTA, INC.) is used. The timing of obtaining the additional information is simultaneous with a timing of measuring the pulse wave, and additionally, may be any timing depending on the usage. The additional information may be input, for example, by the user via the input unit 108 or the like, and any method for obtaining the additional information is employable.

The comprehensive evaluation result indicates the result of evaluating the health condition of the user. As the comprehensive evaluation result, in addition to "healthy" and "unhealthy," a character string indicating the degree of athletic ability relating to the health condition of each user, such as "athletic ability is high" and "athletic ability is low" may be used, and for example, a value such as a difference from any reference value and a deviation value may be used. The comprehensive evaluation result may include, for example, at least any of the evaluation result and the additional information.

The comprehensive evaluation result indicates, for example, a publicly known threshold such as an anaerobic threshold different for each user, and additionally, may indicate, for example, a change over time of the parameter affecting the athletic ability such as a degree of anaerobic metabolism. Examples of the parameter affecting the athletic ability include values indicating the feature regarding the blood composition such as an oxygen uptake, a value of blood carbon dioxide partial pressure (PaCO₂), a dissolved concentration of blood carbon dioxide, a concentration of bicarbonate (HCO₃⁻) contained in blood, and a pH of blood. By including such a parameter in the additional information, the comprehensive evaluation result can be generated with high accuracy.

Especially, since the blood glucose level possibly depends on the exercise condition, the evaluation using the above-described additional information allows attempting the accuracy improvement in the evaluation.

In the aggregating step S150, for example, the aggregation unit refers to a preliminarily set threshold, and generates a result of comparing the evaluation result and the additional information with the threshold as the comprehensive evaluation result. For example, when the evaluation result and the additional information are lower than the threshold, the aggregation unit generates the comprehensive evaluation result indicating that the athletic ability is high, and when the evaluation result and the additional information are higher than the threshold, the aggregation unit generates the comprehensive evaluation result indicating that the athletic ability is low.

The aggregation unit generates, for example, a value in which the evaluation result has been corrected based on the contents of the additional information as the comprehensive evaluation result using a preliminarily set function. For example, the blood glucose spike information included in the evaluation result depends on the exercise condition of the user at the evaluation, and for example, the peak intensity of the blood glucose spike tends to decrease in inverse proportion to the quantity of exercise. Therefore, by correcting the value of the blood glucose spike information based on the contents of the additional information, the factors relative to the change of the blood glucose level can be reduced, thus allowing the evaluation of the user's health condition with high accuracy.

The aggregation unit generates the comprehensive evaluation result by referring to, for example, the data format that is preliminarily stored in the storage unit 104 or the like and recognizable by the user.

The aggregation unit may generate the appropriate comprehensive evaluation result for the evaluation result and the additional information by referring to, for example, a post-processing database. The post-processing database is stored in, for example, the storage unit 104.

In the post-processing database, for example, similarly to the above-described database, post-processing classification information for generating the comprehensive evaluation result for the evaluation result and the additional information may be stored. In the post-processing database, one or more pieces of the post-processing classification information are stored, and additionally, for example, a plurality of post-processing training data used for generating the post-processing classification information may be stored.

The post-processing classification information is, for example, a function indicating a correlation between preliminarily obtained past evaluation results and additional information (post-processing input data) and post-processing reference data associated with the post-processing input data. The post-processing reference data indicates the evaluation result of the athletic ability of the user. The post-processing classification information is generated using a plurality of post-processing training data each of which is a pair of the post-processing input data and the post-processing reference data.

The post-processing classification information indicates a calibration model generated based on a result of an analysis performed by, for example, the above-described regression analysis having the post-processing input data as an explanatory variable and the post-processing reference data as an objective variable. For the post-processing classification information, for example, the calibration model (post-processing calibration model) can be regularly updated, and additionally, for example, may be generated for each piece of the additional information. The post-processing classification information may include, similarly to the above-described classification information, for example, a trained model (post-processing trained model) generated by machine learning using a plurality of post-processing training data.

According to this embodiment, in addition to the effect of the above-described embodiment, the aggregation unit generates the comprehensive evaluation result evaluating the user's health condition based on the evaluation result and the additional information. Therefore, in addition to the evaluation result, the comprehensive evaluation considering the feature regarding the biometric information of the user can be achieved. Accordingly, the health condition based on the change of the blood glucose level of the user can be evaluated.

According to this embodiment, the additional information indicates at least any of the pulse rate, the respiratory rate, the blood pressure, the feature of the blood carbon dioxide, the lactate level, and the oxygen saturation. Therefore, the evaluation based on the plurality of pieces of biometric information can be performed. Accordingly, the improvement of the evaluation accuracy can be attempted.

### (Second Embodiment: Modification of Biometric Information Computing System 100)

Next, a modification of the biometric information computing system 100 in the second embodiment will be described. The difference between the example of the above-described embodiment and the modification is that the additional information is used in calculating the blood glucose level. For the contents similar to the above-described embodiment, the explanation will be omitted.

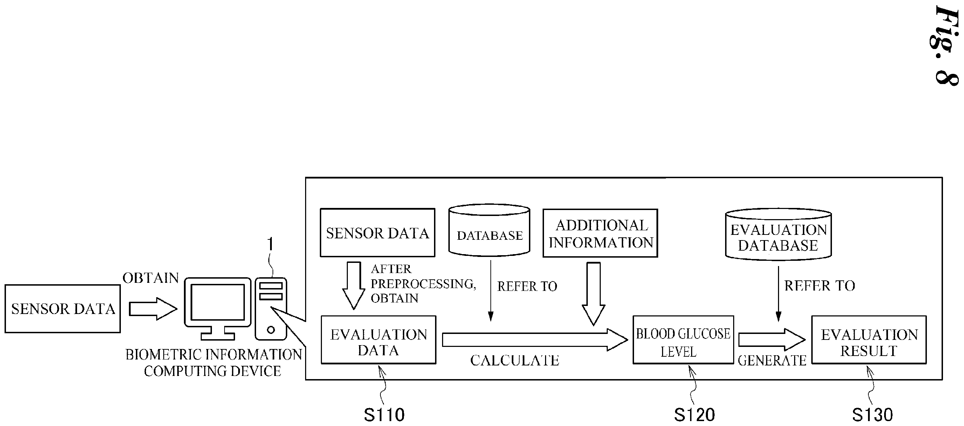

In this modification, for example, as illustrated in Fig. 8, the calculating step S120 includes obtaining the additional information and calculating the blood glucose level based on the evaluation data and the additional information.

The additional information is similar to the above-described contents, and for example, input by the user via the input unit 108 or the like and obtained by the calculating unit 12 or the like. The calculating unit 12 may determine the computing method to the evaluation data, for example, corresponding to the contents of the additional information. In this case, functions or the like different between the kinds of the additional information are stored in the storage unit 104 or the evaluation database. The calculating unit 12 may calculate the blood glucose level, for example, based on the information in which the evaluation data is combined with the additional information.

According to this modification, the calculating unit 12 refers to the database, and calculates the blood glucose level based on the evaluation data and the additional information. Therefore, the parameter effective for calculating the user's blood glucose level can be used. Accordingly, the further improvement of the accuracy in calculating the blood glucose level can be attempted.

### (Third Embodiment: Biometric Information Computing System 100)

Next, an example of a biometric information computing system 100 according to the third embodiment will be described. The difference between the above-described embodiments and the third embodiment is that additional data for generating additional information is obtained. For the contents similar to the above-described embodiment, the explanation will be omitted.

In the biometric information computing system 100 according to this embodiment, the obtaining step S110 includes obtaining the additional data. In the biometric information computing system 100 according to this embodiment, the calculating step S120 includes generating the additional information based on the additional data.

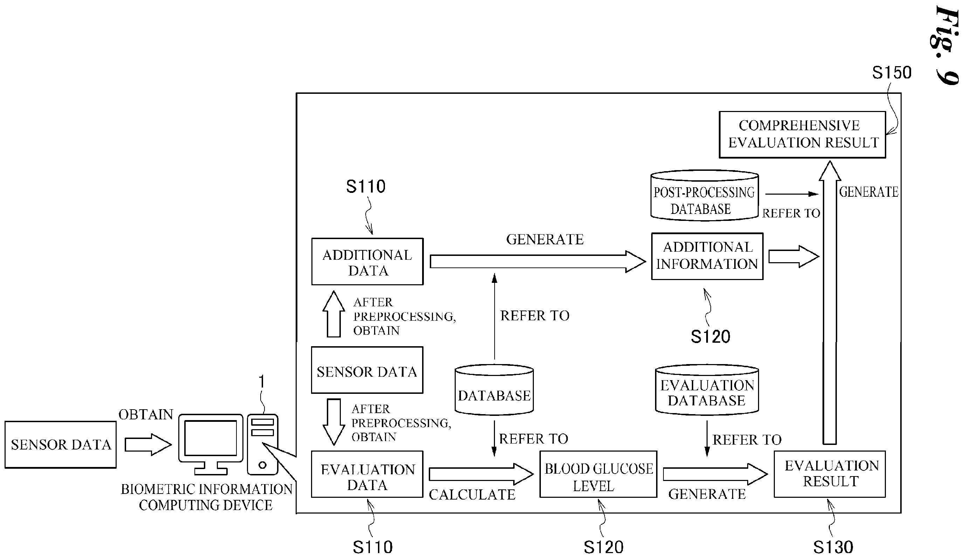

A biometric information computing device 1 according to this embodiment, for example, as illustrated in Fig. 9, performs two kinds of processes on one sensor data generated based on the pulse wave. This causes, for example, the obtaining unit 11 to obtain the evaluation data and the additional data indicating the feature different from the evaluation data based on the same pulse wave. A plurality of additional data may be obtained.

For example, the calculating unit 12 generates the additional information based on the additional data. In this embodiment, the additional information includes the biometric information that can be calculated from the sensor data among the above-described additional information. Corresponding to the kind of the additional information to be generated, for example, the contents of the preprocessing or the like used in obtaining the additional data may be changed.

The calculating unit 12 may, for example, refer to the database and generate the additional information from the additional data. In the database, for example, additional classification information for generating the additional information for the additional data may be stored. The database stores one or more pieces of the additional classification information, and additionally, may store, for example, a plurality of additional training data used for generating the additional classification information.

The additional classification information is, for example, a function indicating a correlation between preliminarily obtained past additional data (additional input data) and additional reference data associated with the additional input data. The additional reference data includes the above-described additional information. The additional classification information is generated using a plurality of additional training data each of which is a pair of the additional input data and the additional reference data.

The additional classification information indicates a calibration model generated based on a result of an analysis performed by, for example, the above-described regression analysis having the additional input data as an explanatory variable and the additional reference data as an objective variable. For the additional classification information, for example, the calibration model (additional calibration model) can be regularly updated, and additionally, may include a plurality of additional calibration models generated for the respective pieces of attribute information such as a gender, an age, and an exercise content of the user. The additional classification information may include, similarly to the above-described classification information, for example, a trained model (additional trained model) generated by machine learning using a plurality of additional training data.

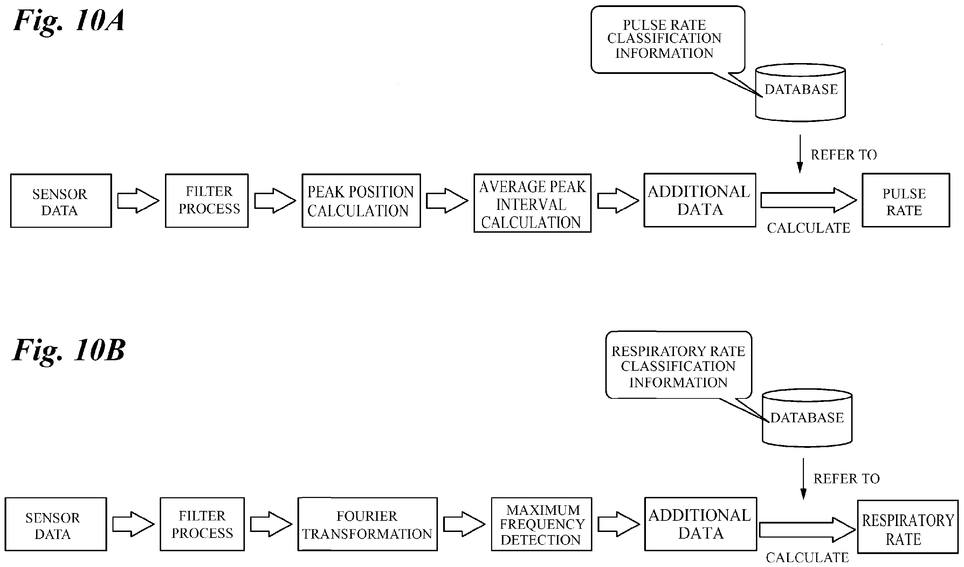

For example, as illustrated in Fig. 10A and Fig. 10B, the content of the preprocessing performed in the obtaining step S110 and the content of the additional classification information referred in the calculating step S120 differ in some cases. The following describes examples of the calculation with the pulse rate and the respiratory rate as the additional information.

### <Pulse Rate Calculation>

For example, when the pulse rate is calculated, as illustrated in Fig. 10A, the obtaining unit 11 performs the above-described filter process on the sensor data and extracts the pulse wave data.

The obtaining unit 11 performs a peak position calculating process on the pulse wave data. In the peak position calculating process, a plurality of peaks (maximum values of amplitudes) included in the pulse wave data are detected and the order of being sampled (corresponding to the time since the start of measurement) is identified. Thus, the obtaining unit 11 obtains peak position data included in the pulse wave data.

Then, the obtaining unit 11 performs an average peak interval calculating process on the peak position data. The average peak interval calculating process calculates peak intervals (the difference between the adjacent orders in which the peaks are sampled) included in the peak position data to calculate, for example, an average value of the peak intervals. Then, the obtaining unit 11 divides the peak intervals or the average value of the peak intervals by a sampling rate of the sensor data and obtains the data indicating the peak intervals corresponding to seconds as the additional data.

Then, the calculating unit 12 refers to the database and calculates the pulse rate relative to the additional data. In this respect, the calculating unit 12 refers to pulse rate classification information among the additional classification information stored in the database. The pulse rate classification information indicates a function that divides, for example, 60 [second] by the peak intervals. Therefore, the calculating unit 12 can calculate, for example, the pulse rate (= 71 [bpm]) relative to the additional data (the peak interval = 0.85 [seconds]). This allows the calculating unit 12 to generate the additional information including the pulse rate.

### <Respiratory Rate Calculation>

For example, when the respiratory rate is calculated, as illustrated in Fig. 10B, the obtaining unit 11 performs the above-described filter process on the sensor data to extract the pulse wave data.

Then, the obtaining unit 11 performs Fourier transform processing on the pulse wave data. In the Fourier transform processing, for example, the pulse wave data indicating sampling period to amplitude is converted into frequency data indicating frequency to intensity. This causes the obtaining unit 11 to obtain the frequency data relative to the pulse wave data.

Then, the obtaining unit 11 performs a maximum frequency detecting process on the frequency data. In the maximum frequency detecting process, the frequency of the maximum intensity between 0.15 Hz to 0.35 Hz in the frequency data is identified. This causes the obtaining unit 11 to obtain the value of the identified frequency as the additional data.

Then, the calculating unit 12 refers to the database and calculates the respiratory rate relative to the additional data. In this respect, the calculating unit 12 refers to respiratory rate classification information among the additional classification information stored in the database. The respiratory rate classification information indicates, for example, a function that multiplies the identified frequency by 60 [second]. Therefore, the calculating unit 12 can calculate, for example, the respiratory rate (= 13.5 [bpm]) relative to the additional data (the identified frequency = 0.225 Hz). Thus, the calculating unit 12 can generate, for example, the additional information including the respiratory rate.

Thus, the biometric information computing device 1 can set the preprocessing on the sensor data depending on the content of the data included in the additional information and appropriately set the content of the database to be referred to.

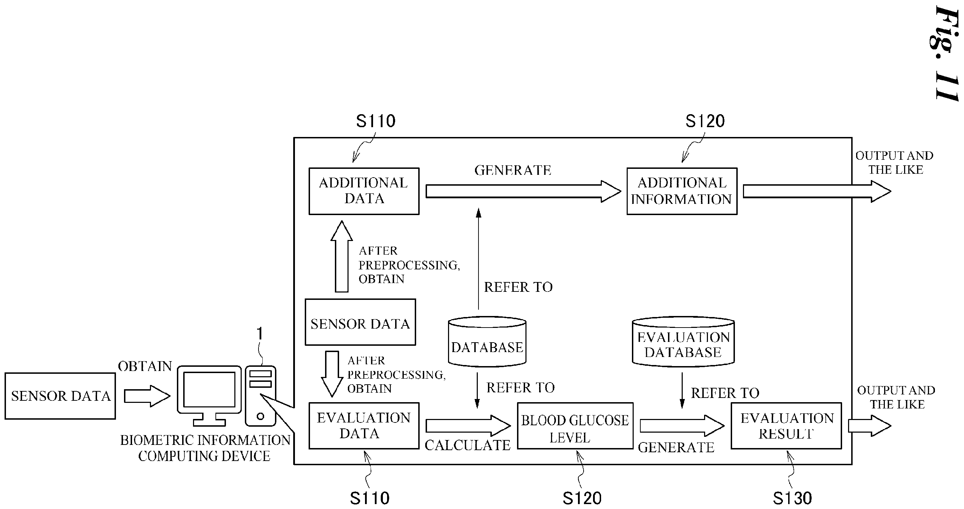

In the biometric information computing system 100 according to this embodiment, for example, as illustrated in Fig. 11, the output unit 14 may output the generated additional information. In this case, since the additional information generated by referring to the information different from the evaluation result can be used, the user and the like can make a multifaceted determination. Accordingly, the appropriate evaluation corresponding to the request from the user can be easily achieved.

According to this embodiment, in addition to the effects of the above-described embodiments, the obtaining unit 11 obtains the additional data indicating the feature different from the evaluation data based on the pulse wave. The calculating unit 12 generates the additional information based on the additional data. That is, the evaluation result and the additional information are generated based on one pulse wave. In view of this, by using a plurality of kinds of information based on the same parameter, the comprehensive calculation or evaluation based on the multifaceted aspect can be achieved.

### (Fourth Embodiment: Biometric Information Computing System 100)

Next, an example of a biometric information computing system 100 in the fourth embodiment will be described. The difference between the above-described embodiments and the fourth embodiment is that the above-described additional information is obtained in the evaluating step S130. For the contents similar to the above-described embodiments, the explanation will be omitted.

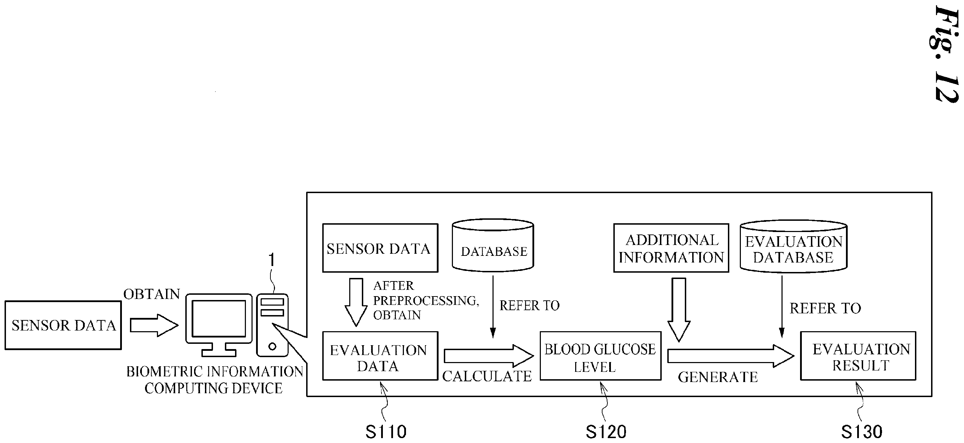

In the biometric information computing system 100 according to this embodiment, for example, as illustrated in Fig. 12, the evaluating step S130 includes obtaining the additional information and generating the evaluation result based on the plurality of blood glucose levels and the additional information.

The additional information is similar to the above-described contents, and for example, input by the user via the input unit 108 or the like and obtained by the evaluation unit 13 or the like. For example, the evaluation unit 13 may obtain the additional information generated by the calculating unit 12 based on the additional data obtained by the obtaining unit 11.

The evaluation unit 13 may determine the computing method for the plurality of blood glucose levels, for example, corresponding to the contents of the additional information. In this case, the functions or the like different for respective kinds of the additional information are stored in the storage unit 104 or the database. The evaluation unit 13 may generate the evaluation result, for example, based on information in which the plurality of blood glucose levels are combined with the additional information.

According to this embodiment, in addition to the effects of the above-described embodiments, the evaluation unit 13 includes obtaining the additional information and generating the evaluation result based on the plurality of blood glucose levels and the additional information. Therefore, the multifaceted evaluation result considering the feature of the user in addition to the plurality of blood glucose levels can be generated.

### (Fifth Embodiment: Biometric Information Computing System 100)

Next, an example of a biometric information computing system 100 in the fifth embodiment will be described. The difference between the above-described embodiments and the fifth embodiment is that attribute-based classification information appropriate for the evaluation data is selected from a plurality of pieces of the attribute-based classification information included in the classification information. For the contents similar to the above-described embodiments, the explanation will be omitted.

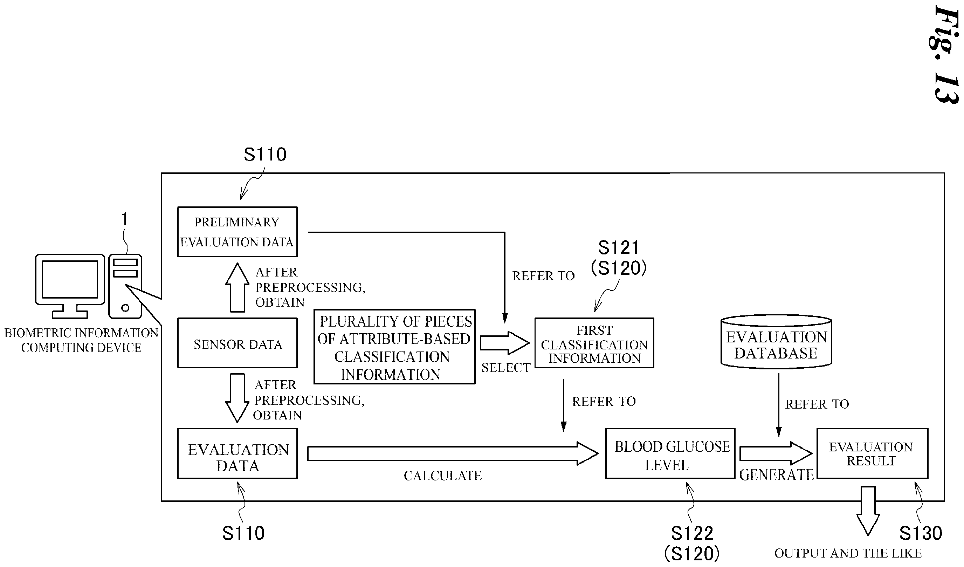

In a biometric information computing device 1 according to this embodiment, for example, as illustrated in Fig. 13, the calculating step S120 includes a selecting step S121 and an attribute-based calculating step S122.

The selecting step S121 refers to preliminary evaluation data, and selects specific attribute-based classification information (for example, first classification information) among a plurality of pieces of attribute-based classification information. The selecting step S121 can be executed by, for example, a selecting unit included in the calculating unit 12.

The preliminary evaluation data indicates a feature different from that of the evaluation data and indicates, for example, a feature similar to that of the above-described additional data. The preliminary evaluation data may be used for, for example, generating the additional information, similarly to, for example, the additional data described above.

The attribute-based calculating step S122 refers to the selected first classification information, and calculates the blood glucose level for the evaluation data. The attribute-based calculating step S122 can be executed by, for example, an attribute-based calculating unit included in the calculating unit 12.

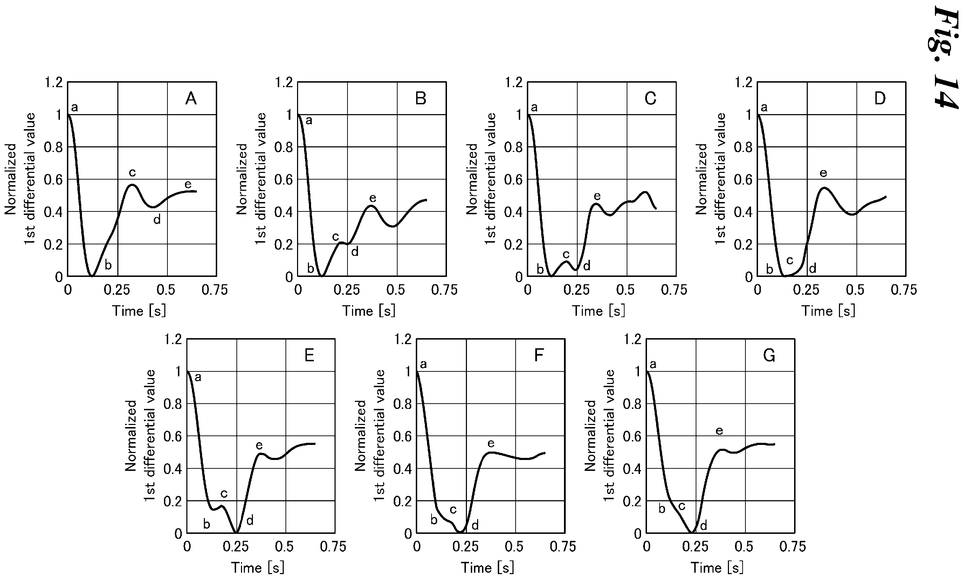

The plurality of pieces of attribute-based classification information are calculated using mutually different training data. For example, when data corresponding to the acceleration pulse wave of the examinee is used as the input data of the training data, for example, the input data is prepared for each of seven kinds (A to G) as illustrated in Fig. 14, thus generating seven kinds of attribute classification information.

When the plurality of pieces of attribute-based classification information are stored in the database, for example, the obtaining unit 11 obtains the evaluation data corresponding to the acceleration pulse wave of the user and the preliminary evaluation data. Then, the calculating unit 12 refers to the preliminary evaluation data, and selects the first classification information. Subsequently, the calculating unit 12 refers to the first classification information, and calculates the blood glucose level for the evaluation data. Therefore, among the pieces of the attribute classification information, the optimal classification information for the user can be selected.

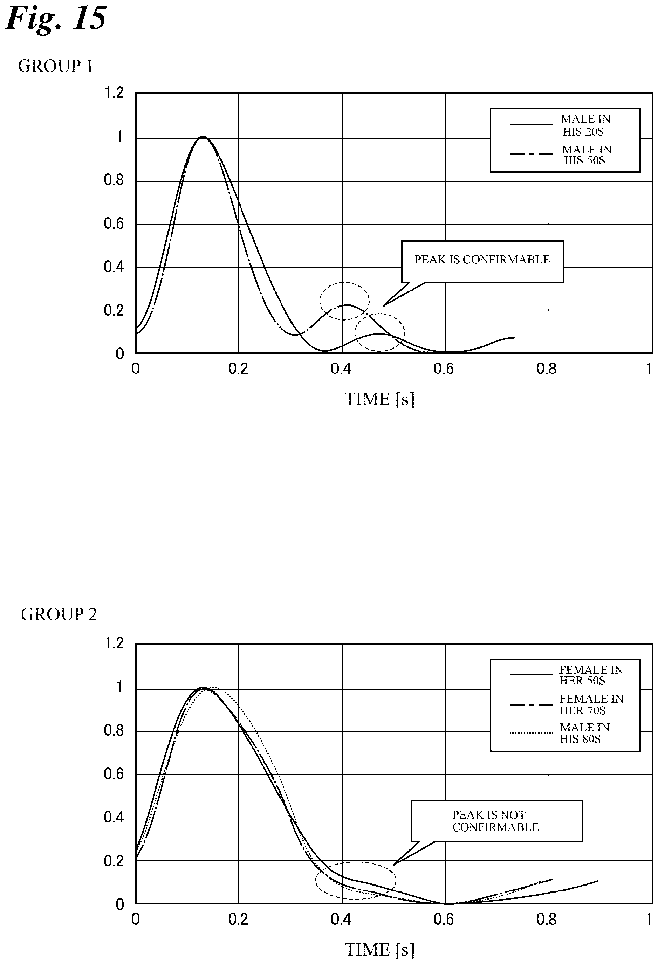

For example, when data corresponding to the velocity pulse wave of the examinee is used as the input data of the training data, for example, the input data may be prepared for each of two kinds (group 1, group 2) as illustrated in Fig. 15, and two kinds of attribute classification information may be generated.

Here, while the data corresponding to the acceleration pulse wave illustrated in Fig. 14 is easily classified in detail based on the feature, in calculating the feature regarding the blood composition, the reduction in accuracy due to the false detection of the peak or the like is concerned. While the data corresponding to the velocity pulse wave illustrated in Fig. 15 has a difficulty in detailed classification based on the feature compared with the data corresponding to the acceleration pulse wave, the blood glucose level can be calculated with high accuracy because of the less false detection of the peak or the like.

Based on the above-described circumstances, the plurality of pieces of attribute classification information may include, for example, the data corresponding to the acceleration pulse wave as illustrated in Fig. 14 as selection data used for selecting the specific classification information, and for the training data in generating the attribute classification information, the data corresponding to the velocity pulse wave may be used.

In this case, as the obtaining step S110, for example, the obtaining unit 11 obtains data corresponding to the velocity pulse wave from the sensor data based on the user's pulse wave as the evaluation data. The obtaining unit 11 obtains data corresponding to the acceleration pulse wave from the sensor data as the preliminary evaluation data.

Next, as the selecting step S121, for example, the calculating unit 12 refers to the preliminary evaluation data, identifies selection data (first selection data) most similar to the preliminary evaluation data among a plurality of selection data including the data corresponding to the acceleration pulse wave, and selects first classification information associated with the first selection data. Then, as the attribute-based calculating step S122, the calculating unit 12 refers to the first classification information, and calculates the blood glucose level for the evaluation data. This allows attempting the further improvement of the evaluation accuracy.

Here, an example of the data used for the above-described selection data or the like will be described.

For example, as illustrated in Fig. 14, the acceleration pulse wave includes inflection points of ***a*** to ***e**.* For example, when the normalization is performed with a point ***a*** as the maximum peak in the acceleration pulse wave, the inflection points of a point *b,* a point c, a point *d,* and a point e in the order from the point ***a**,* the point ***a*** set to 1, and the point *b* or the point *d* as the minimum value set to 0, the acceleration pulse wave can be classified into seven patterns using a method of classifying with the values of the respective inflection points and a magnitude relationship of differences of the values. First, when the values of the inflection points meet ***b < d**,* the acceleration pulse wave is classified into a pattern A or B. In the case of ***b < d*** and further c ≥ 0.5, the acceleration pulse wave is classified into the pattern A, and otherwise the pattern B. Next, when the values of the inflection points meet *b* ≈ *d,* the acceleration pulse wave is classified into a pattern C or D. In the case of ***b*** ≈ ***d*** and further c ≈ 0, the acceleration pulse wave is classified into the pattern D, and otherwise the pattern C. Finally, in the case of ***b > d**,* the acceleration pulse wave can be classified into any of a pattern E, F, or G. The acceleration pulse wave is classified into the pattern E in the case of *b* **>** *d* and further *b* **<** *c,* the pattern F in the case of b ≈ c, and the pattern G in the case of ***b > c.***

For example, the calculating unit 12 determines, for example, which pattern of Fig. 14 the preliminary evaluation data corresponds to, and identifies the first selection data. For example, when the input inflection point *b* of the preliminary evaluation data is smaller than the inflection point ***d*** and further the inflection point ***c*** ≥ 0.5 is met, the pattern A is identified as the first selection data. Accordingly, the blood glucose level can be accurately calculated by referring to the classification information appropriate for the feature of the evaluation data.

According to this embodiment, in addition to the effects of the above-described embodiments, the calculating unit 12 includes the selecting unit that selects the first classification information by referring to the preliminary evaluation data, and the attribute-based calculating unit that calculates the blood glucose level for the evaluation data by referring to the first classification information. Therefore, the optimal attribute classification information for the feature of the pulse wave is selected, and then, the blood glucose level for the evaluation data can be calculated. This allows attempting the further improvement of the evaluation accuracy.

According to this embodiment, the obtaining unit 11 obtains the data corresponding to the velocity pulse wave based on the pulse wave as the evaluation data. The obtaining unit 11 obtains the data corresponding to the acceleration pulse wave based on the pulse wave as the preliminary evaluation data. Therefore, the attribute classification information can be selected using the acceleration pulse wave that facilitates the classification of the feature of the pulse wave compared with the velocity pulse wave. Additionally, the blood glucose level can be calculated using the velocity pulse wave that facilitates the calculation of the blood glucose level compared with the acceleration pulse wave. This allows attempting the further improvement of the evaluation accuracy.

### (Fifth Embodiment: Modification of Biometric Information Computing System 100)

Next, a modification of the biometric information computing system 100 in the fifth embodiment will be described. The difference between the above-described example of the embodiment and this modification is that first classification information is selected based on the additional information. For the contents similar to the above-described embodiments, the explanation will be omitted.

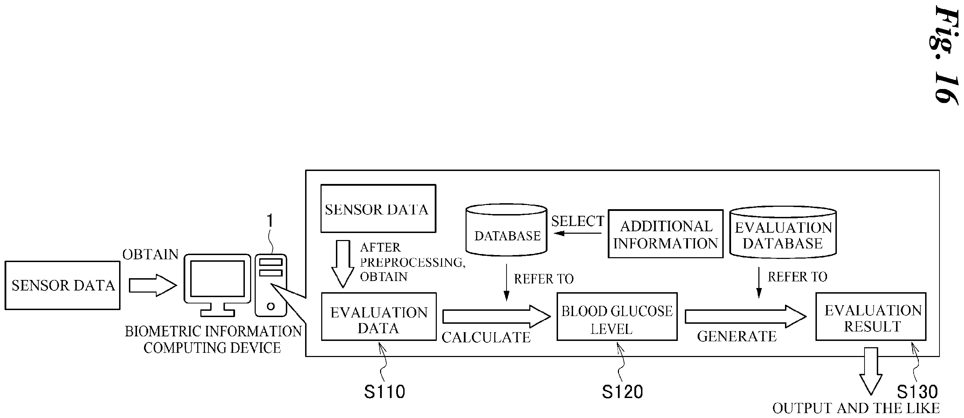

In this modification, for example, as illustrated in Fig. 16, the calculating step S120 obtains the additional information. The calculating step S120 selects the first classification information based on the additional information among the classification information, and calculates the blood glucose level for the evaluation data by referring to the first classification information.

For example, the calculating unit 12 obtains the additional information similar to that of the above-described embodiment. The calculating unit 12 may obtain, for example, the additional information generated based on the above-described additional data.

For example, the classification information includes a plurality of functions or calibration models preliminarily assigned to the respective features of the additional information. In this case, the calculating unit 12 can select the function or the like (for example, first classification information) appropriate for the feature of the additional information among the classification information. As the method for selecting the first classification information, a publicly known technique may be used.

According to this modification, the calculating unit 12 includes referring to the first classification information selected based on the additional information and calculating the blood glucose level for the evaluation data. Therefore, the optimal classification information for the feature of the user is selected, and then the blood glucose level for the evaluation data can be calculated. This allows attempting the further improvement of the evaluation accuracy.

### (Sixth Embodiment: Biometric Information Computing System 100)

Next, an example of a biometric information computing system 100 in the sixth embodiment will be described. The difference between the above-described embodiments and the sixth embodiment is that the health condition of the user is indicated as a status of the user. For the contents similar to the above-described embodiments, the explanation will be omitted.

The biometric information computing system 100 according to this embodiment is used for generating the evaluation result on the health condition of the user. The evaluation result on the user's health condition indicates a result of medical examination of the user, an index indicating the degree of health, an insurance premium corresponding to the user's health condition, or the like.

The biometric information computing system 100 calculates a blood glucose level evaluation result including the blood glucose level from the evaluation data based on the user's pulse wave, and generates the evaluation result from the evaluation data and the blood glucose level evaluation result.

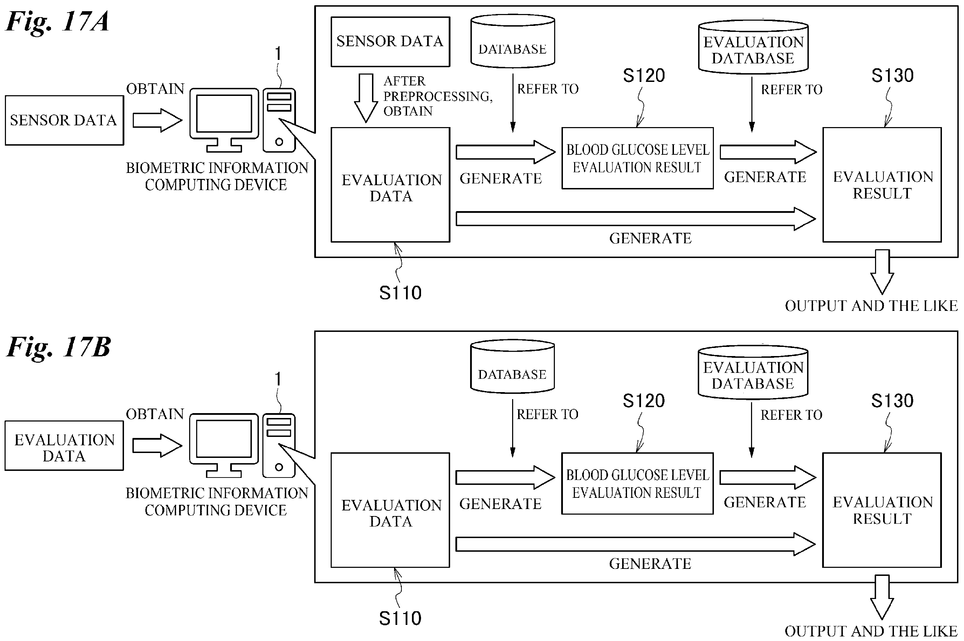

In the biometric information computing system 100, for example, as illustrated in Fig. 17A, the biometric information computing device 1 obtains the sensor data generated by the sensor 5 or the like. Then, the biometric information computing device 1 performs preprocessing such as a filter process on the obtained sensor data, thus obtaining the evaluation data.

The biometric information computing device 1 calculates the blood glucose level evaluation result for the evaluation data, and generates the evaluation result for the evaluation data and the blood glucose level evaluation result. Therefore, the evaluation result can be generated from the evaluation data and the blood glucose level evaluation result obtained based on the feature of the pulse wave. This allows generating the evaluation result of the user with high accuracy.

The biometric information computing device 1 refers to the database in generating the blood glucose level evaluation result for the evaluation data. The database stores, similarly to the above-described embodiment, the classification information generated using a plurality of training data.

For example, as illustrated in Fig. 3A, the classification information is generated using a plurality of training data each of which is a pair of input data based on a training pulse wave obtained in the past and reference data including the blood glucose level associated with the input data. Therefore, in the generation of the blood glucose level evaluation result, the blood glucose level can be quantitatively included based on the input data proven in the past and the output data. This allows attempting the accuracy improvement in generating the evaluation result.

The biometric information computing device 1 outputs, for example, the generated evaluation result to a display or the like.

In the biometric information computing system 100, for example, as illustrated in Fig. 17B, the evaluation data may be obtained from the sensor 5 or the like. In this case, the preprocessing of obtaining the evaluation data from the sensor data is performed by the sensor 5 or the like.

The evaluation data indicates data for generating the blood glucose level evaluation result and the evaluation result.

The database is used mainly for generating the evaluation result for the evaluation data. The database stores one or more pieces of the classification information, and additionally, may store, for example, the plurality of training data used for generating the classification information. The classification information is, for example, a function indicating a correlation between preliminarily obtained past evaluation data (input data) and reference data including the blood glucose level, and the explanation will be omitted because details are similar to the above-described embodiment.

The classification information is, similarly to the above-described embodiment, for example, a function indicating a correlation between preliminarily obtained past evaluation data (input data) and reference data including the blood glucose level.

### <Blood Glucose Level Evaluation Result>

The blood glucose level evaluation result is generated as data of the same kind as the reference data, and includes the blood glucose level. The blood glucose level evaluation result is generated as data the same as or similar to the reference data by referring to the classification information. The blood information includes the blood glucose level evaluation result.

In the biometric information computing system 100, for example, a plurality of evaluation data are obtained along any time series, and a plurality of blood glucose levels evaluation results for the respective evaluation data are generated. In the biometric information computing system 100, for example, a plurality of evaluation data may be obtained at any timings such as a change of the quantity of exercise, and a plurality of blood glucose levels evaluation results for the respective evaluation data may be generated.

The evaluation result indicates information on the health condition of the user. The evaluation result includes, for example, a plurality of blood glucose level evaluation results and the evaluation data, and additionally, may include a difference value in a pair of the evaluation data and the blood glucose level evaluation result or a publicly known threshold derived based on a plurality of evaluation data and the blood glucose level evaluation result. By outputting the evaluation result, the user's health condition can be obtained.

The obtaining unit 11 of the biometric information computing device 1 according to this embodiment may obtain, for example, information on an evaluation target input via the input unit 108 or the like by the user, such as a feature, an exercise content, an athletic event, and the like of the user, and include the information in the evaluation data. The information on the evaluation target may be used in generating, for example, the blood glucose level evaluation result or the evaluation result.

The calculating unit 12 refers to the database, and calculates the blood glucose level evaluation result for the evaluation data. The calculating unit 12 refers to, for example, the classification information stored in the database, calculates the blood glucose level for the evaluation data, and calculates it as the blood glucose level evaluation result. The calculating unit 12 calculates a plurality of blood glucose level evaluation results for the mutually different evaluation data.

The evaluation unit 13 generates the evaluation result based on the evaluation data and the blood glucose level evaluation result generated from the evaluation data. The evaluation unit 13 generates the evaluation result transformed into a format in which the user can understand the health condition, for example, using a display format preliminarily stored in the storage unit 104 or the like.

The evaluation unit 13 may generate, for example, the evaluation result from the evaluation data and the blood glucose level evaluation result by referring to the evaluation database.

The evaluation database may store, for example, similarly to the above-described database, evaluation classification information for generating the evaluation result for the evaluation data and the blood glucose level evaluation result. The evaluation database stores one or more pieces of the evaluation classification information, and additionally, may store, for example, a plurality of evaluation training data used for generating the evaluation classification information.

The evaluation classification information is, for example, a function indicating a correlation between preliminarily obtained past evaluation data and blood glucose level evaluation result (evaluation input data) and evaluation reference data associated with the evaluation input data.

### (Sixth Embodiment: Operation of Biometric Information Computing System 100)

Next, an example of the operation of the biometric information computing system 100 according to the embodiment will be described. For the contents similar to the above-described embodiments, the explanation will be omitted.

The calculating step S120 refers to the database, and generates the blood glucose level evaluation result including the blood glucose level for the evaluation data. For example, the calculating unit 12 refers to the classification information, and calculates the blood glucose level for the evaluation data. The calculating unit 12 generates the blood glucose level evaluation result including the calculated value. The calculating unit 12 stores the generated blood glucose level evaluation result in the storage unit 104, for example, via the storing unit 15. The blood glucose level indicates a specific value, and additionally, for example, an error range (for example, "xx ± 5 mg/dl") may be calculated.

The evaluating step S130 generates the evaluation result based on the evaluation data and the blood glucose level evaluation result generated from the evaluation data. For example, the evaluation unit 13 obtains the evaluation data and the blood glucose level evaluation result generated by the calculating unit 12. The evaluation unit 13 generates the evaluation result from the evaluation data and the blood glucose level evaluation result using a preliminarily set function or the like, and additionally, for example, may generate the evaluation result by referring to the above-described evaluation database.

The evaluation unit 13 generates the evaluation result by, for example, combining the blood glucose level evaluation result with the evaluation data. In this case, the evaluation result may be generated by referring to the evaluation database in addition to using a preliminarily set function or the like. Accordingly, the feature of the user's pulse wave can be easily taken into consideration, and the accuracy of the evaluation result can be improved.

Here, in the waveform signal of the pulse wave, the value of blood glucose level generatable from the waveform signal of the pulse wave has variations due to the attribute such as a gender and an age of the user in some cases. For example, when a case where the pulse wave measured from a male in his 20s as a user is processed is compared with a case where the pulse wave measured from a female in her 50s as a user is processed, it is concerned that variations occur in the accuracy of the generatable blood glucose level depending on the processing method. That is, for obtaining the evaluation result on the user's health condition with sufficient accuracy using the blood glucose level generated from the waveform signal of the pulse wave, it is necessary to perform a process corresponding to the feature of the user.

On the other hand, in the conventional technique, it is not assumed to generate the evaluation result on the user's health condition for the blood glucose level generated from the waveform signal of the pulse wave through the process corresponding to the feature of the user. Therefore, in the conventional technique, since the process corresponding to the feature of the user is not assumed, it is concerned that the evaluation result on the user's health condition cannot be generated with high accuracy.

In contrast, according to this embodiment, the calculating unit 12 generates the blood glucose level evaluation result including the blood glucose level for the evaluation data. The evaluation unit 13 generates the evaluation result on the user's health condition based on the evaluation data and the blood glucose level evaluation result generated from the evaluation data. Therefore, the evaluation result can be generated from the evaluation data and the blood glucose level evaluation result obtained based on the feature of the pulse wave. This allows generating the evaluation result of the user with high accuracy.

According to this embodiment, the calculating unit 12 refers to the database, and generates the blood glucose level evaluation result for the evaluation data. The database stores the classification information calculated using a plurality of training data. Therefore, in generating the evaluation result, the quantitative blood glucose level based on data proven in the past can be included. This allows attempting the accuracy improvement in generating the evaluation result.

According to this embodiment, the classification information is a calibration model obtained using the PLS regression analysis having the input data as the explanatory variable and the reference data as the objective variable. Therefore, in the comparison with the case of calculating the classification information using machine learning or the like, the number of the training data can be substantially reduced, and the calibration model can be easily updated. Accordingly, the facilitation of establishing and updating the biometric information computing system 100 can be attempted.

### (Seventh Embodiment: Biometric Information Computing System 100)

Next, an example of a biometric information computing system 100 in the seventh embodiment will be described. The difference between the above-described embodiments and the seventh embodiment is that a function for generating the evaluation result from the blood glucose level evaluation result is selected based on the feature of the evaluation data, and the evaluation result is generated from the blood glucose level evaluation result. For the contents similar to the above-described embodiments, the explanation will be omitted.

The evaluation unit 13 may determine the computing method for the blood glucose level evaluation result, for example, corresponding to the feature of the evaluation data. In this case, functions or the like for generating the evaluation result from the blood glucose evaluation result different for respective features of the evaluation data are stored in, for example, the evaluation database stored in the storage unit 104 or the like. The evaluation unit 13 may, for example, select the function stored in the storage unit 104 based on the feature such as an intensity and a shape of the peak of the evaluation data, and generate the evaluation result from the blood glucose level evaluation result. The evaluation unit 13 may, for example, select the evaluation classification information appropriate for the evaluation data as the above-described function from a plurality of pieces of evaluation classification information based on the feature of the evaluation data.

For example, when data corresponding to the acceleration pulse wave of the user is used as the evaluation data, the "feature of the evaluation data" may be determined based on which classification pattern of the seven kinds as illustrated in Fig. 14 the data belongs to. For example, when data corresponding to the velocity pulse wave of the user is used as the evaluation data, the "feature of the peak of the evaluation data" may be determined based on which classification pattern of the two kinds as illustrated in Fig. 15 the data belongs to.

The evaluation database may store, for example, similarly to the above-described database, the evaluation classification information for generating the evaluation result for the blood glucose level evaluation result. The evaluation database stores one or more pieces of the evaluation classification information, and additionally, may store, for example, a plurality of evaluation training data used for generating the evaluation classification information.

The evaluation classification information is, for example, a function indicating a correlation between the blood glucose level evaluation result (evaluation input data) associated with the preliminarily obtained past evaluation data and evaluation reference data associated with the evaluation input data. The evaluation reference data indicates the evaluation result of the user. The evaluation classification information is generated using a plurality of evaluation training data each of which is a pair of the evaluation input data and the evaluation reference data.

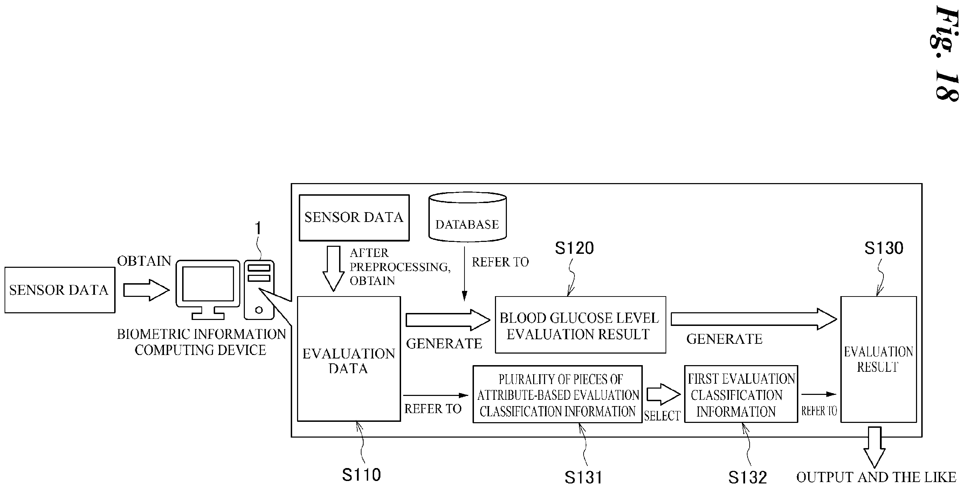

In the biometric information computing system 100 according to this embodiment, for example, as illustrated in Fig. 18, the evaluating step S130 includes an evaluation selecting step S131 and an attribute-based evaluating step S132.

The evaluation selecting step S131 refers to the evaluation data, and selects specific evaluation classification information (for example, first evaluation classification information) among a plurality of pieces of attribute-based evaluation classification information. The evaluation selecting step S131 can be executed by, for example, an evaluation selecting unit included in the evaluation unit 13.

The attribute-based evaluating step S132 refers to the selected first evaluation classification information, and generates the evaluation result for the blood glucose level evaluation result. The attribute-based evaluating step S132 can be executed by, for example, an attribute-based evaluation unit included in the evaluation unit 13.

The plurality of pieces of attribute-based evaluation classification information include mutually different functions for generating the evaluation result from the blood glucose level evaluation result. The plurality of pieces of attribute-based evaluation classification information may be calculated using training data. For example, as input data of the training data, the blood glucose level evaluation results associated with the evaluation data of the above-described respective classification patterns are used. In this case, for example, the input data are prepared for the respective blood glucose level evaluation results associated with the evaluation data of the seven kinds of classification patterns as illustrated in Fig. 14, and the seven kinds of attribute-based evaluation classification information are generated.

When the plurality of pieces of attribute-based evaluation classification information are stored in the database, for example, the obtaining unit 11 obtains the evaluation data corresponding to the acceleration pulse wave of the user. Then, the evaluation unit 13 refers to the feature of the peak of the evaluation data, and selects the first evaluation classification information. Subsequently, the evaluation unit 13 refers to the first evaluation classification information, and generates the evaluation result for the blood glucose level evaluation result. Accordingly, the optimal evaluation classification information for the user can be selected among the plurality of pieces of attribute-based evaluation classification information.

For example, when the data corresponding to the velocity pulse wave of the examinee is used as the input data of the training data, for example, the input data may be prepared for the respective blood glucose level evaluation results associated with the evaluation data of the two kinds of classification patterns as illustrated in Fig. 15, and the two kinds of attribute-based evaluation classification information may be generated. This allows attempting the further improvement of the evaluation accuracy.

According to this embodiment, in addition to the effects of the above-described embodiments, the evaluation unit 13 selects the function for generating the evaluation result from the blood glucose level evaluation result based on the feature of the evaluation data. Therefore, the appropriate function can be selected corresponding to the feature of the evaluation data different for each user. This allows generating the evaluation result of the user with high accuracy.

### (Eighth Embodiment: Biometric Information Computing System 100)

Next, an example of a biometric information computing system 100 in the eighth embodiment will be described. The difference between the above-described embodiments and the eighth embodiment is that an aggregating step S150 is included. For the contents similar to the above-described embodiments, the explanation will be omitted.

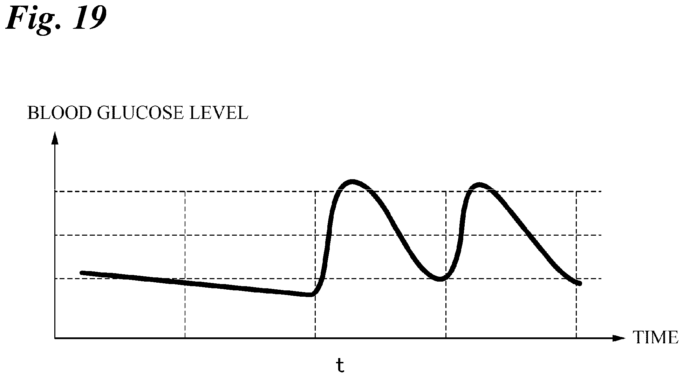

In the aggregating step S150 of the biometric information computing system 100 according to this embodiment, for example, the above-described evaluation unit 13 generates a blood glucose trend based on the plurality of blood glucose level evaluation results generated from the mutually different evaluation data. The evaluation unit 13 generates the evaluation result from the evaluation data and the blood glucose trend. The blood glucose trend is, for example, a graph or the like indicating a change of the blood glucose level relative to a time ***t*** as illustrated in Fig. 19. The blood glucose trend may be, for example, one obtained by differentiating or integrating the graph indicating the change of the blood glucose level relative to the time ***t*** by the time ***t**.* The blood glucose trend may include the blood glucose level spike indicating the local maximum point of the blood glucose level. The blood glucose trend may include a value of HbA1c.

The evaluation unit 13, for example, obtains the plurality of blood glucose level evaluation results generated from the mutually different evaluation data, and obtains the blood glucose trend regarding the trend of the user's blood glucose level from the time series change in the blood glucose level evaluation results.

The evaluation unit 13 may, for example, refer to the evaluation database and generate the evaluation result from the evaluation data and the blood glucose trend.

The evaluation database may store, for example, similarly to the above-described database, evaluation classification information for generating the evaluation result for the evaluation data and the blood glucose trend. The evaluation database stores one or more pieces of the evaluation classification information, and additionally, may store, for example, a plurality of evaluation training data used for generating the evaluation classification information.

The evaluation classification information is, for example, a function indicating a correlation between preliminarily obtained past evaluation data and blood glucose trend (evaluation input data) and evaluation reference data associated with the evaluation input data. The evaluation reference data indicates the evaluation result of the user. The evaluation classification information is generated using a plurality of evaluation training data each of which is a pair of the evaluation input data and the evaluation reference data.

According to this embodiment, in addition to the effects of the above-described embodiments, the evaluation S130 obtains the blood glucose trend regarding the trend of the user's blood glucose level from the time series change in the plurality of blood glucose level evaluation results generated from the mutually different evaluation data, and generates the evaluation result from the blood glucose trend and the evaluation data. Therefore, the feature of the blood glucose level different for each user, such as a blood glucose level spike, can be easily obtained. Accordingly, the evaluation result of the use can be generated with high accuracy.

### (Eighth Embodiment: Operation of Biometric Information Computing System 100)

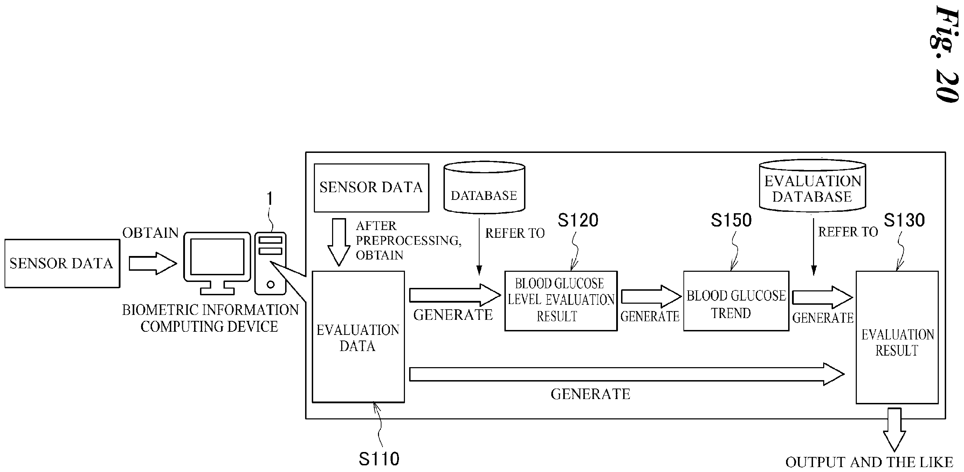

Next, an example of the operation of the biometric information computing system 100 according to the embodiment will be described. Fig. 20 is a flowchart illustrating an example of the operation of the biometric information computing system 100 in this embodiment. In the operation of the biometric information computing system 100 according to this embodiment, as illustrated in Fig. 20, the evaluating step S130 includes an aggregating step S150.

### <Aggregating Step S150>

The aggregating step S150 obtains, for example, a plurality of blood glucose level evaluation results generated from the mutually different evaluation data, and obtains a blood glucose trend regarding the trend of the user's blood glucose level from the time series change in the blood glucose level evaluation results. The aggregating step S150 can be achieved by, for example, the evaluation unit 13 included in the biometric information computing device 1.

### <Evaluating Step S130>

Next, the evaluating step S130 generates the evaluation result based on the evaluation data and the blood glucose trend. For example, the evaluation unit 13 obtains the evaluation data and the blood glucose trend generated by the calculating unit 12. The evaluation unit 13 generates the evaluation result from the evaluation data and the blood glucose trend using a preliminarily set function or the like, and additionally, for example, may generate the evaluation result by referring to the above-described evaluation database. Therefore, the feature of the blood glucose level different for each user, such as a blood glucose level spike, can be easily obtained. Accordingly, the evaluation result of the use can be generated with high accuracy.

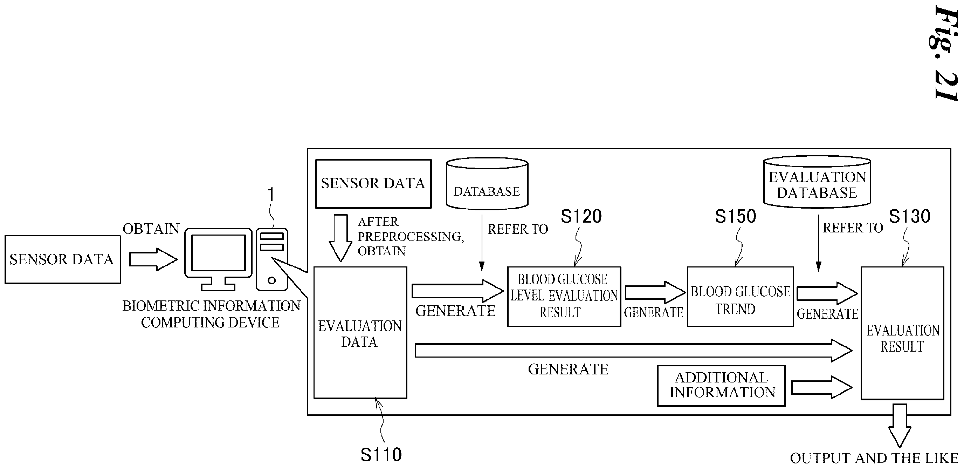

For example, as illustrated in Fig. 21, the evaluating step S130 obtains the above-described additional information, and generates the evaluation result based on the evaluation data, the blood glucose level evaluation result, and the additional information. The evaluating step S130 may include the aggregating step S150. In this case, the evaluating step S130 obtains the additional information, and generates the evaluation result based on the evaluation data, the blood glucose trend, and the additional information.

The evaluation unit 13 may, for example, refer to the evaluation database and generate the evaluation result appropriate for the evaluation data, the blood glucose level evaluation result, and the additional information.

The evaluation classification information is, for example, a function indicating a correlation between preliminarily obtained past evaluation data, blood glucose level evaluation result, and additional information (evaluation input data) and evaluation reference data associated with the evaluation input data. The evaluation reference data indicates the evaluation result of the user. The evaluation classification information is generated using a plurality of evaluation training data each of which is a pair of the evaluation input data and the evaluation reference data.

According to this embodiment, in addition to the effects of the above-described embodiments, the evaluation unit 13 includes obtaining the additional information indicating the feature of the user and generating the evaluation result based on the evaluation data, the blood glucose level evaluation result, and the additional information. Therefore, by using a plurality of kinds of information, the comprehensive evaluation based on the multifaceted aspect can be achieved. Accordingly, the evaluation result of the user can be generated with higher accuracy.

### (Ninth Embodiment: Biometric Information Computing System 100)

Next, an example of a biometric information computing system 100 in the ninth embodiment will be described. The difference between the above-described embodiments and the ninth embodiment is that the above-described additional information is obtained in the calculating step S120 and the blood glucose level evaluation result is generated based on the evaluation data and the additional information. For the contents similar to the above-described embodiments, the explanation will be omitted.

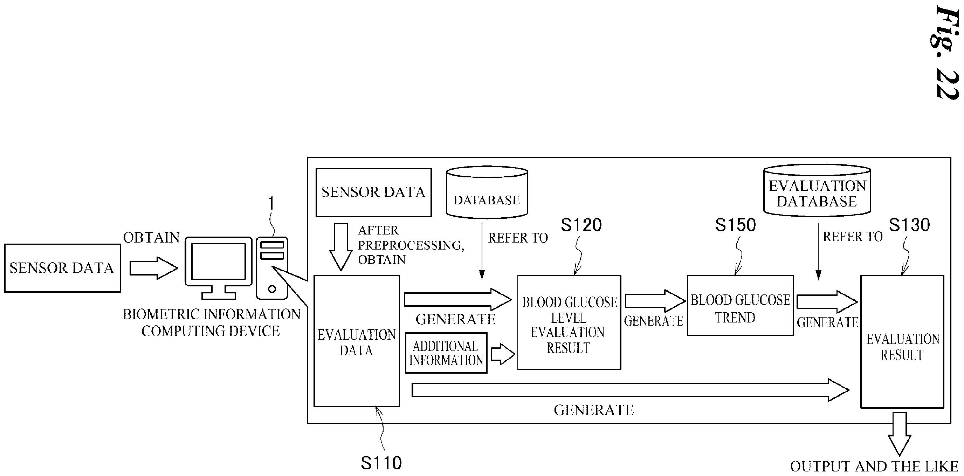

In the biometric information computing system 100 according to this embodiment, for example, as illustrated in Fig. 22, the calculating step S120 includes obtaining the additional information and generating the blood glucose level evaluation result based on the evaluation data and the additional information. The evaluating step S130 may include the aggregating step S150. In this case, the evaluating step S130 generates the evaluation result based on the evaluation data and the blood glucose trend.

The additional information is similar to the above-described contents, and for example, input by the user via the input unit 108 or the like and obtained by the calculating unit 12 or the like. For example, the calculating unit 12 may obtain the additional information generated by the calculating unit 12 based on the additional data obtained by the obtaining unit 11.

The calculating unit 12 may determine the computing method for the blood glucose level evaluation result, for example, corresponding to the contents of the additional information. In this case, the functions or the like different for respective kinds of the additional information are stored in the storage unit 104 or the evaluation database. The calculating unit 12 may generate the blood glucose level evaluation result, for example, based on information in which the evaluation data is combined with the additional information.

According to this embodiment, in addition to the effects of the above-described embodiments, the calculating unit 12 includes obtaining the additional information and generating the blood glucose level evaluation result based on the evaluation data and the additional information. Therefore, the comprehensive evaluation based on the multifaceted aspect can be achieved using the parameter having the correlation with the blood glucose level evaluation result of the user. Accordingly, the evaluation result of the user can be generated with higher accuracy.

### (Tenth Embodiment: Biometric Information Computing System 100)

Next, an example of a biometric information computing system 100 in the tenth embodiment will be described. The difference between the above-described embodiments and the tenth embodiment is that the classification information appropriate for the evaluation data is selected from a plurality of pieces of classification information based on the additional information. For the contents similar to the above-described embodiments, the explanation will be omitted.

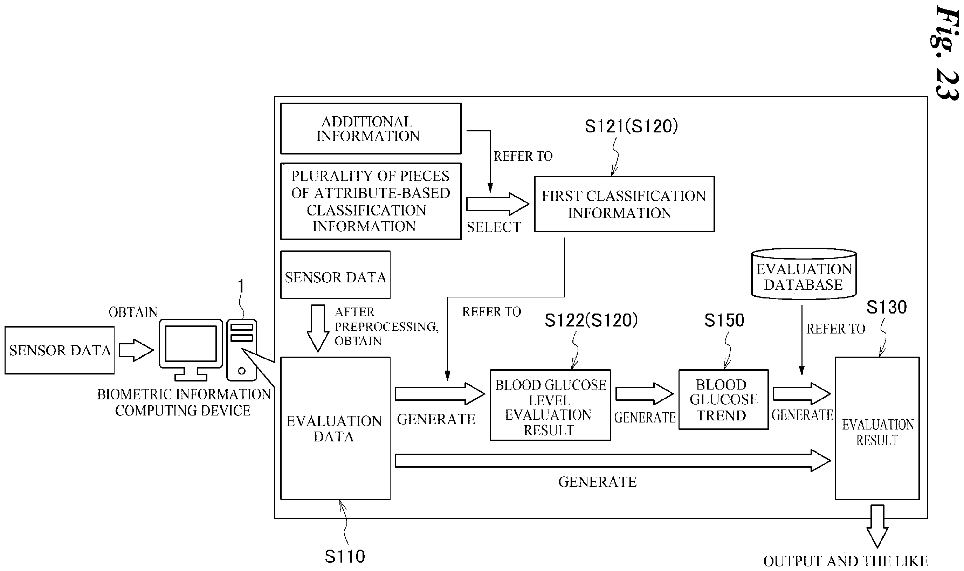

In a biometric information computing device 1 according to this embodiment, for example, as illustrated in Fig. 23, the calculating step S120 includes a selecting step S121 and an attribute-based calculating step S122. The evaluating step S130 may include an aggregating step S150. In this case, the evaluating step S130 generates the evaluation result based on the evaluation data and the blood glucose trend.

The selecting step S121 refers to the additional information, and selects specific classification information (for example, first classification information) among a plurality of pieces of attribute-based classification information. The selecting step S121 can be executed by, for example, a selecting unit included in the calculating unit 12.

In the attribute-based calculating step S122, the biometric information computing device 1 refers to the selected first classification information, calculates the blood glucose level for the evaluation data, and generates the blood glucose level evaluation result. The attribute-based calculating step S122 can be executed by, for example, an attribute-based generating unit included in the calculating unit 12.

The plurality of pieces of attribute-based classification information are calculated using mutually different training data. For example, as input data of the training data, the input data are prepared for the respective features of the additional information, thus generating the plurality of pieces of attribute-based classification information. For example, when the additional information is the age of the user, the input data may be prepared for each age group of 10 to 30 years old, 30 to 50 years old, 50 to 70 years old, and the like.

When the plurality of pieces of attribute-based classification information are stored in the database, for example, the obtaining unit 11 obtains the evaluation data and the additional information. Then, the calculating unit 12 refers to the additional information, and selects the first classification information. Subsequently, the calculating unit 12 refers to the first classification information, and generates the blood glucose level evaluation result for the evaluation data. Therefore, the optimal classification information for the user can be selected among the plurality of pieces of attribute-based classification information.

In this case, as the obtaining step S110, for example, the obtaining unit 11 obtains the evaluation data of the user and the age of the user as the additional information.

Next, as the selecting step S121, for example, the calculating unit 12 refers to the additional information, and selects the first classification information associated with the additional information. Then, as the attribute-based calculating step S122, the calculating unit 12 refers to the first classification information, and generates the blood glucose level evaluation result for the evaluation data. This allows attempting the further improvement of the evaluation accuracy.

According to this embodiment, in addition to the effects of the above-described embodiments, the calculating unit 12 includes the selecting unit that refers to the additional information and selects the first classification information, and the attribute-based generating unit that refers to the first classification information and generates the blood glucose level evaluation result for the evaluation data. Therefore, the optimal attribute-based classification information is selected for the additional information, and then the blood glucose level evaluation result for the evaluation data can be generated. This allows attempting the further improvement of the evaluation accuracy.

### (Eleventh Embodiment: Biometric Information Computing System 100)

Next, an example of a biometric information computing system 100 in the eleventh embodiment will be described. The difference between the above-described embodiments and the eleventh embodiment is that blood information includes the feature regarding the blood composition and an athletic ability of the user is indicates as the status of the user. For the contents similar to the above-described embodiments, the explanation will be omitted.

The biometric information computing system 100 according to this embodiment is used for generating information on the athletic ability of the user. The information on the athletic ability of the user indicates, for example, a trend of the athletic ability of the user, a degree of difference from a specific reference value, or the like.

The trend of the athletic ability indicates, for example, a publicly known threshold different for each user, such as an anaerobic threshold, and additionally, indicates, for example, a change over time of a parameter affecting the athletic ability, such as a degree of anaerobic metabolism. Examples of the parameter affecting the athletic ability include values indicating the feature regarding the blood composition such as an oxygen uptake, a value of blood carbon dioxide partial pressure (PaCO₂), a dissolved concentration of blood carbon dioxide, a concentration of bicarbonate (HCO₃⁻) contained in blood, and a pH of blood.

The biometric information computing system 100 generates the blood information including the feature regarding the blood composition from the evaluation data based on the user's pulse wave. The biometric information computing system 100 generates, for example, the evaluation result indicating the trend of the athletic ability of the user based on a plurality of pieces of blood information generated in time series.

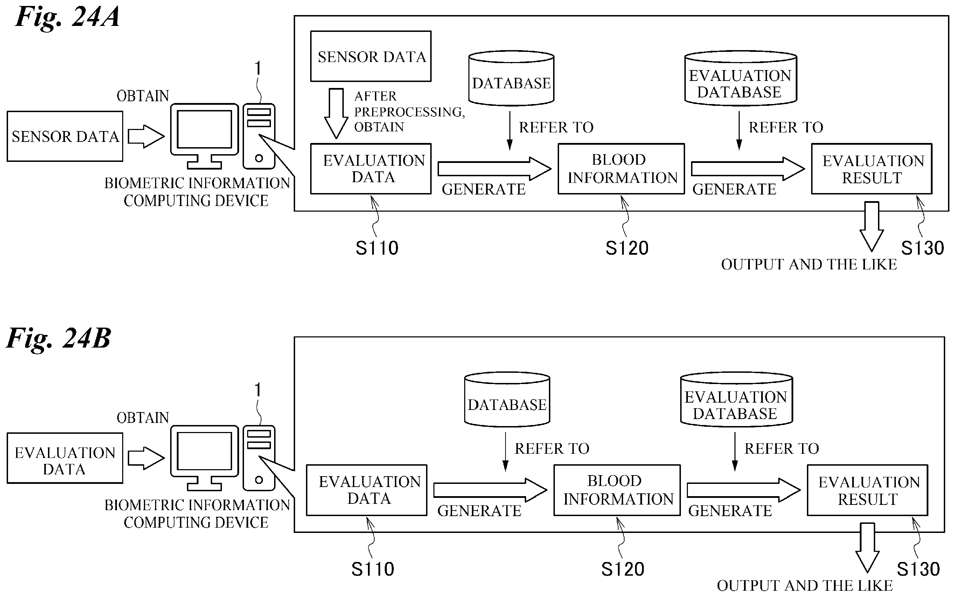

In the biometric information computing system 100, for example, as illustrated in Fig. 24A, a biometric information computing device 1 obtains the sensor data generated by the sensor 5 or the like. Then, the biometric information computing device 1 performs preprocessing such as a filter process on the obtained sensor data, thus obtaining the evaluation data.

The biometric information computing device 1 generates the blood information for the evaluation data. Then, the biometric information computing device 1 generates the evaluation result based on a plurality of pieces of blood information generated from mutually different evaluation data. Therefore, the evaluation result can be directly generated from the feature regarding the blood composition regardless of the influence of the evaluation target such as an individual feature, an exercise content, and an athletic event. This allows generating the information on the athletic ability of the user with high accuracy.

Here, the biometric information computing device 1 refers to the database in generating the blood information for the evaluation data. The database stores the classification information generated using a plurality of training data.

For example, as illustrated in Fig. 3A, the classification information is generated using a plurality of training data each of which is a pair of input data based on a training pulse wave obtained in the past and reference data including the feature regarding the blood composition associated with the input data. Therefore, in the generation of the blood information, the feature regarding the blood composition can be quantitatively included based on the input data proven in the past and the output data. This allows attempting the accuracy improvement in the generation of the blood information.

The biometric information computing device 1 outputs the generated evaluation result to, for example, a display or the like. The evaluation result includes information indicating the trend of the athletic ability of the user by a numerical value, and includes, for example, a threshold different for each user such as an anaerobic threshold. The evaluation result may include, for example, information indicative of the evaluation on the trend of the athletic ability of the user, such as "during aerobic exercise" and "during anaerobic exercise."

In the biometric information computing system 100, for example, as illustrated in Fig. 24B, the evaluation data may be obtained from the sensor 5 or the like. In this case, the preprocessing of obtaining the evaluation data from the sensor data is performed by the sensor 5 or the like.

The evaluation data indicates data for generating the blood information.

The database is mainly used for generating the blood information for the evaluation data. The database stores one or more pieces of the classification information, and additionally, may store, for example, the plurality of training data used for generating the classification information. The classification information is a function indicating, for example, a correlation between preliminarily obtained past evaluation data (input data) and reference data including the feature regarding the blood composition, and the explanation will be omitted because details are similar to the above-described embodiment.

The reference data includes the feature regarding the blood composition of the examinee measured using a measuring device or the like. For example, when the sensor 5 or the like is worn by the examinee to generate the training sensor data, by measuring the feature of blood carbon dioxide or the like of the examinee, the reference data associated with the input data can be obtained. In this case, while a timing of measuring the feature of blood carbon dioxide or the like is preferably simultaneous with a timing of generating the training sensor data, the timing of measuring the feature of blood carbon dioxide or the like may be a timing, for example, before or after about one to 10 minutes.

The "feature regarding the blood composition" indicates measurable concentration, proportion, or the like of the compositions in blood, and indicates, for example, at least any of the feature of the blood carbon dioxide concentration, the blood lactate level, and the oxygen saturation. The "feature of blood carbon dioxide" indicates a level of carbon dioxide contained in blood. As the feature of blood carbon dioxide, for example, besides a value of a blood carbon dioxide partial pressure (PaCO₂) being used, a dissolved concentration of blood carbon dioxide and a concentration of bicarbonate (HCO₃⁻) contained in blood may be used or a value considering pH of blood may be used depending on the situation.

The blood information is generated as data of the same kind as the reference data, and includes the feature regarding the blood composition. The blood information is generated as data the same as or similar to the reference data by referring to the classification information. In the biometric information computing system 100, for example, a plurality of evaluation data are obtained along any time series, and a plurality of pieces of blood information for the respective evaluation data are generated. In the biometric information computing system 100, for example, a plurality of evaluation data may be obtained at any timings such as a change of the quantity of exercise, and a plurality of pieces of blood information for the respective evaluation data may be generated.

The evaluation result indicates the trend of the athletic ability of the user. The evaluation result includes, for example, a plurality of pieces of blood information, and additionally, may include a difference value in a pair of the blood information or a publicly known threshold derived based on the plurality of pieces of blood information. By outputting the evaluation result, the user's athletic ability can be obtained.

The obtaining unit 11 of the biometric information computing device 1 according to this embodiment may obtain, for example, information on an evaluation target input via the input unit 108 or the like by the user, such as a feature, an exercise content, an athletic event, and the like of the user, and include the information in the evaluation data. The information on the evaluation target may be used in generating, for example, the blood information or the evaluation result.

The calculating unit 12 refers to the database, and generates the blood information for the evaluation data. The calculating unit 12 refers to, for example, the classification information stored in the database, calculates the value of the blood carbon dioxide partial pressure or the like for the evaluation data, and generates it as the blood information. The calculating unit 12 generates a plurality of pieces of blood information for the mutually different evaluation data.

The evaluation unit 13 generates the evaluation result based on the plurality of pieces of blood information generated from the mutually different evaluation data. The evaluation unit 13 generates the evaluation result transformed into a format in which the user can understand the trend of the athletic ability, for example, using a display format preliminarily stored in the storage unit 104 or the like.

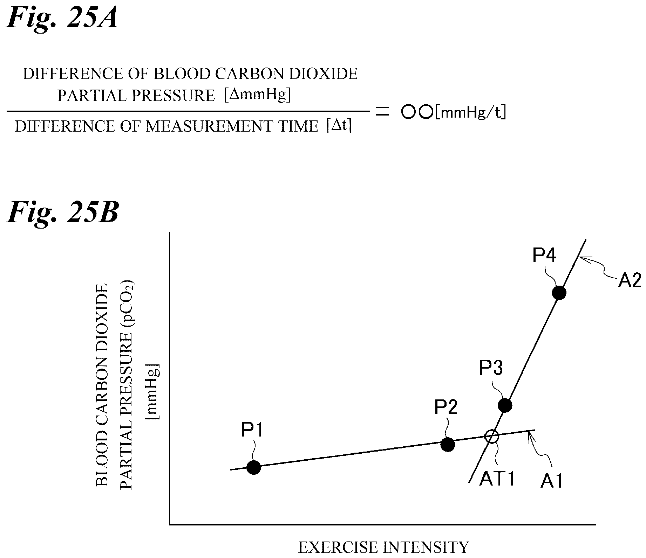

For example, as illustrated in Fig. 25A, the evaluation unit 13 calculates a value obtained by dividing a difference between two pieces of blood information (in Fig. 25A, blood carbon dioxide partial pressure is illustrated as an example) by a difference of the measurement time as a degree of anaerobic metabolism, and generates the evaluation result including the degree of anaerobic metabolism. In this case, the condition of anaerobic exercise different for each user can be easily obtained. For example, the evaluation unit 13 may refer to the reference value preliminarily stored in the storage unit 104, generate the evaluation result indicating the fact of being in the aerobic exercise state when the degree of anaerobic metabolism is smaller than the reference value, and generate the evaluation result indicating the fact of being in the anaerobic exercise state when the degree of anaerobic metabolism is equal to or more than the reference value.

The evaluation unit 13 may generate, for example, the evaluation result from the plurality of pieces of blood information by referring to the evaluation database.

The evaluation database may store, for example, similarly to the above-described database, evaluation classification information for generating the evaluation result for the plurality of pieces of blood information. The evaluation database stores one or more pieces of the evaluation classification information, and additionally, may store, for example, a plurality of evaluation training data used for generating the evaluation classification information.

The evaluation classification information is, for example, a function indicating a correlation between a preliminarily obtained plurality of pieces of past blood information (evaluation input data) and evaluation reference data associated with the evaluation input data. The evaluation reference data indicates the trend of the athletic ability of the user. The evaluation classification information is generated using a plurality of evaluation training data each of which is a pair of the evaluation input data and the evaluation reference data.

For example, as illustrated in Fig. 25B, the evaluation database may store functions A1, A2 indicating relations between the exercise intensity and the blood information (in Fig. 25B, blood carbon dioxide partial pressure is illustrated as an example). The function A1 indicates, for example, the relation between the exercise intensity during the aerobic exercise and the blood carbon dioxide partial pressure, and the function A2 indicates, for example, the relation between the exercise intensity during the anaerobic exercise and the blood carbon dioxide partial pressure. The functions A1, A2 may include a plurality of functions.

For example, when the calculating unit 12 generates a first partial pressure P1 and a second partial pressure P2 as the plurality of pieces of blood information, the evaluation unit 13 refers to the function A1, and generates the evaluation result indicating "during aerobic exercise" based on the partial pressures P1, P2.

For example, when the calculating unit 12 generates a third partial pressure P3 and a fourth partial pressure P4 as the plurality of pieces of blood information, the evaluation unit 13 refers to the function A2, and generates the evaluation result indicating "during anaerobic exercise" based on the partial pressures P3, P4.

For example, when the calculating unit 12 generates the first partial pressure P1, the second partial pressure P2, the third partial pressure P3, and the fourth partial pressure P4 as the plurality of pieces of blood information, the evaluation unit 13 refers to the functions A1, A2, and generates the evaluation result indicating an anaerobic threshold AT1 based on the partial pressures P1, P2, P3, and P4.

### (Eleventh Embodiment: Operation of Biometric Information Computing System 100)

Next, an example of the operation of the biometric information computing system 100 according to the embodiment will be described. For the contents similar to the above-described embodiments, the explanation will be omitted.

The calculating step S120 refers to the database, and generates the blood information including the feature regarding the blood composition (for example, value of the blood carbon dioxide partial pressure) for the evaluation data. For example, the calculating unit 12 refers to the classification information, and calculates the value of the blood carbon dioxide partial pressure for the evaluation data. The calculating unit 12 calculates the blood information including the calculated value. The calculating unit 12 stores the generated blood information in the storage unit 104, for example, via the storing unit 15. The value of the blood carbon dioxide partial pressure or the like indicates a specific value, and additionally, for example, an error range (for example, "xx ± 2 mmHg") may be calculated.

The evaluating step S130 generates the evaluation result based on the plurality of pieces of blood information generated from the mutually different evaluation data. For example, the evaluation unit 13 obtains the plurality of pieces of blood information generated by the calculating unit 12. The evaluation unit 13 generates the evaluation result from the plurality of pieces of blood information using a preliminarily set function or the like, and additionally, for example, may generate the evaluation result by referring to the above-described evaluation database.

For example, when the blood information indicates the value of the blood carbon dioxide partial pressure, the evaluation unit 13 can generate the evaluation result including the degree of anaerobic metabolism from the time series change in the plurality of pieces of blood information. Therefore, the condition of anaerobic exercise different for each user can be easily obtained.

Here, in the evaluation of the athletic ability of the user, a threshold such as an anaerobic threshold (AT: Anaerobic Threshold), a heart rate threshold (HRT: Heart Rate Threshold), or a lactate threshold (LT: Lactate Threshold) is used in some cases. It has been known that these thresholds can be estimated using the pulse rate. However, the evaluation method using the pulse rate remains only roughly obtaining the trend due to the influence of the evaluation target such as an individual feature, an exercise content, and an athletic event, and appropriately estimating the above-described threshold is difficult. In view of this, in the conventional technique, it is concerned that the information on the athletic ability of the user cannot be generated with high accuracy.

In contrast, according to this embodiment, the calculating unit 12 generates the blood information including the feature regarding the blood composition for the evaluation data. The evaluation unit 13 generates the evaluation result indicating the trend of the athletic ability of the user based on the plurality of pieces of blood information generated from the mutually different evaluation data. Therefore, the evaluation result can be directly generated from the feature regarding the blood composition regardless of the influence of the evaluation target such as an individual feature, an exercise content, and an athletic event. This allows generating the information on the athletic ability of the user with high accuracy.

According to this embodiment, the calculating unit 12 refers to the database, and generates the blood information for the evaluation data. The database stores the classification information calculated using a plurality of training data. Therefore, in the generation of the blood information, the quantitative feature regarding the blood composition can be included based on the data proven in the past. This allows attempting the accuracy improvement in generating the blood information.

According to this embodiment, the evaluation unit 13 generates the evaluation result including the degree of anaerobic metabolism from the time series change in the plurality of pieces of blood information. Therefore, the condition of the anaerobic exercise different for each user can be easily obtained. This allows keeping the exercise condition corresponding to the athletic ability of the user.

According to this embodiment, the classification information is a calibration model obtained using the PLS regression analysis having the input data as the explanatory variable and the reference data as the objective variable. Therefore, in the comparison with the case of calculating the classification information using machine learning or the like, the number of the training data can be substantially reduced, and the calibration model can be easily updated. Accordingly, the facilitation of establishing and updating the biometric information computing system 100 can be attempted.

### (Twelfth Embodiment: Biometric Information Computing System 100)

Next, an example of a biometric information computing system 100 in the twelfth embodiment will be described. The difference between the above-described embodiments and the twelfth embodiment is that additional information is used and an aggregating step S150 is included. For the contents similar to the above-described embodiments, the explanation will be omitted.

In the biometric information computing system 100 according to this embodiment, for example, the aggregating step S150 is performed after the above-described evaluating step S130, and the outputting step S140 is performed after the aggregating step S150.

The aggregating step S150, for example, similarly to the above-described embodiments, obtains the additional information and generates the comprehensive evaluation result based on the evaluation result and the additional information. the aggregating step S150 can be executed by, for example, an aggregation unit included in the evaluation unit 13.

The comprehensive evaluation result indicates the result that evaluates the athletic ability of the user. As the comprehensive evaluation result, besides character strings indicating the degree of athletic ability for each user, such as "high athletic ability" and "low athletic ability," being used, for example, a difference with any reference value and a numerical value, such as a deviation value, may be used.

In the aggregating step S150, for example, the aggregation unit refers to a preliminarily set threshold, and generates the result of comparing the evaluation result and the additional information with the threshold as the comprehensive evaluation result. For example, when the evaluation result and the additional information are lower than the threshold, the aggregation unit generates the comprehensive evaluation result indicating that the athletic ability is high, and when the evaluation result and the additional information are higher than the threshold, the aggregation unit generates the comprehensive evaluation result indicating that the athletic ability is low.

According to this embodiment, in addition to the effects of the above-described embodiments, the aggregation unit generates the comprehensive evaluation result evaluating the user's athletic ability based on the evaluation result and the additional information. Therefore, in addition to the evaluation result, the comprehensive evaluation considering the feature of the user can be achieved. This allows generating the information on the athletic ability of the user with higher accuracy.

According to the above-described embodiments, the calculating step S120 calculates the blood information of the user for the evaluation data. The evaluating step S130 generates the evaluation result regarding the status of the user based on the blood information. Accordingly, the quantitative evaluation on the status of the user can be achieved.

While in the respective embodiments described above, the case where the value of the blood carbon dioxide partial pressure is mainly used as the feature of blood carbon dioxide has been described, the same applies to the case where at least any one of the dissolved concentration of blood carbon dioxide, the concentration of bicarbonate contained in blood, and pH of blood is used, thereby omitting the description. In particular, when the value of the blood carbon dioxide partial pressure is used as the feature of blood carbon dioxide, the reference data tends to be easily collected compared with the other values. Therefore, it is possible to achieve a significant reduction in time and cost spent when the classification information is generated.

The above-described respective embodiments may be performed in any combination depending on the usage.

While the embodiments of the present invention have been described, the embodiments have been presented as examples, and are not intended to limit the scope of the invention. The novel embodiments described herein can be embodied in a variety of other configurations. Various omissions, substitutions and changes can be made without departing from the gist of the invention. The embodiments and the modifications thereof are within the scope and the gist of the invention and within the scope of the inventions described in the claims and their equivalents.

### DESCRIPTION OF REFERENCE SIGNS

- 1:: Biometric information computing device
- 3:: Communications network
- 4:: Server
- 5:: Sensor
- 6:: Detecting unit
- 10:: Housing
- 11:: Obtaining unit
- 12:: Calculating unit
- 13:: Evaluation unit
- 14:: Output unit
- 15:: Storing unit
- 16:: Learning unit
- 50:: Obtaining unit
- 51:: Communication I/F
- 52:: Memory
- 53:: Instruction unit
- 54:: Internal bus
- 55:: Wristband
- 100:: Biometric information computing system
- 101:: CPU
- 102:: ROM
- 103:: RAM
- 104:: Storage unit
- 105:: I/F
- 106:: I/F
- 107:: I/F
- 108:: Input unit
- 109:: Display unit
- 110:: Internal bus
- S110:: Obtaining step
- S120:: Calculating step
- S130:: Evaluating step
- S140:: Outputting step

## Claims

1. A biometric information computing system that calculates blood information regarding blood of a user, comprising:
obtaining means that obtains evaluation data based on a pulse wave of the user;
a database that stores classification information generated using a plurality of training data, the training data being a pair of input data based on a preliminarily obtained training pulse wave and reference data including the blood information associated with the input data;
calculating means that calculates the blood information for the evaluation data by referring to the database; and
evaluation means that generates an evaluation result regarding a status of the user based on the blood information.

2. The biometric information computing system according to claim 1, wherein
the blood information includes a blood glucose level, and
the evaluation means generates the evaluation result including blood glucose spike information indicating a change of the blood glucose level of the user based on a plurality of the blood glucose levels of the user calculated from the mutually different evaluation data.

3. The biometric information computing system according to claim 1, wherein
the blood information includes a blood glucose level, and
the evaluation means generates the evaluation result based on the evaluation data and the blood glucose level calculated from the evaluation data.

4. The biometric information computing system according to claim 1, wherein
the blood information includes a feature regarding a composition of the blood, and
the evaluation means generates the evaluation result indicating a trend of an athletic ability of the user based on a plurality of the features regarding the blood composition calculated from the mutually different evaluation data.

5. The biometric information computing system according to claim 1, comprising
aggregation means that obtains additional information indicating a feature regarding biometric information of the user and generates a comprehensive evaluation result evaluating a health condition of the user based on the evaluation result and the additional information.

6. The biometric information computing system according to claim 1, wherein
the obtaining means includes obtaining additional data indicating a feature different from the evaluation data based on the pulse wave, and
the calculating means includes referring to the database and generating additional information indicating a feature regarding biometric information of the user for the additional data.

7. The biometric information computing system according to claim 1, wherein
the evaluation means includes:
obtaining additional information indicating a feature regarding biometric information of the user; and
generating the evaluation result based on a plurality of the feature data and the additional information.

8. The biometric information computing system according to claim 1, wherein
the obtaining means includes obtaining preliminary evaluation data different from the evaluation data based on the pulse wave,
the classification information includes a plurality of pieces of attribute-based classification information calculated using the mutually different training data, and
the calculating means includes:
selection means that refers to the preliminary evaluation data and selects first classification information among the plurality of pieces of attribute-based classification information; and
attribute-based calculating means that refers to the first classification information and calculates the blood information for the evaluation data.
